# EUROPEAN PATENT SPECIFICATION

(11) **EP 0 393 690 B1**
(45) Date of publication and mention of the grant of the patent: **29.03.1995**
(21) Application number: 90107493.0
(22) Date of filing: 20.04.1990
(51) Int. Cl.: C12N 15/52, C12N 9/00, C12P 23/00, C12P 9/00, C12P 5/00

(54) **DNA sequences useful for the synthesis of carotenoids**
Zur Synthese von Karotinoiden verwendbare DNA-Sequenzen
Séquences de DNA utilisables dans la synthèse de caroténoides

(30) Priority: 21.04.1989 JP 103078/89; 05.03.1990 JP 53255/90
(43) Date of publication of application: 24.10.1990
(73) Proprietor: KIRIN BEER KABUSHIKI KAISHA, Shibuya-ku Tokyo-To (JP)
(72) Inventor: Misawa, Norihiko, c/o Kirin Beer, 3, Miyahara-Cho, Takasaki-Shi, Gunma-Ken (JP); Kobayashi, Kazuo, c/o Kirin Beer, 3, Miyahara-Cho, Takasaki-Shi, Gunma-Ken (JP); Nakamura, Katsumi, c/o Kirin Beer, 3, Miyahara-Cho, Takasaki-Shi, Gunma-Ken (JP)
(74) Representative: Reichel, Wolfgang, Dipl.-Ing.

(56) References cited:
- JOURNAL OF BACTERIOLOGY, vol. 168, no. 2, November 1986, pages 607-612,American Society for Microbiology; K.L. PERRY et al.: "Cloning and regulation of Erwinia herbicola pigment genes"
- J. GEN. MICROBIOL., vol. 130, 1984, pages 1623-1631, SGM; G. THIRY: "Plasmids of the epiphytic bacterium Erwinia uredovora"
- CHEMICAL ABSTRACTS, vol. 109, no. 17, 24th October 1988, page 172, abstract no.143512y, Columbus, Ohio, US; G. GIULIANO et al.: "A genetic-physical map of the Rhodobacter capsulatus carotenoid biosynthesis gene cluster", & MGG, MOL. GEN. GENET. 1988, 213(1), 78-83
- CHEMICAL ABSTRACTS, vol. 110, no. 19, 8th May 1989, page 211, abstract no.167285y, Columbus, Ohio, US; M.A NELSON et al.: "Molecular cloning of a Neurospora crassa carotenoid biosynthetic gene (albino-3) regulated by bluelight and the products of the white collar genes", & MOL. CELL. BIOL. 1989, 9(3), 1271-6
- CHEMICAL ABSTRACTS, vol. 107, no. 7, 17th August 1987, page 222, abstract no.53404u, Columbus, Ohio, US; J.M. PEMBERTON et al.: "Expression of Rhodopseudomonas sphaeroides carotenoid photopigment genes in phylogenetically related nonphotosynthetic bacteria", & CURR. MICROBIOL. 1987, 15(2), 67-71

## Description

### Field of the Art

The present invention relates to DNA sequences which are useful for the synthesis of carotenoids such as lycopene, β-carotene, zeaxanthin or zeaxanthin-diglucoside.

The present invention also relates to processes for producing such carotenoid compounds.

### Related Art

Carotenoids are distributed widely in green plants. They are yellow-orange-red lipids which are also present in some mold, yeast and so forth, and have recently received increased attention as natural coloring materials for foods. Among these carotenoids, β-carotene is a typical one, which is used as a coloring materials and as a precursor of vitamin A in mammals as well. It is also examined for its use as a component for preventing cancer [see, for example, SHOKUHIN TO KAIHATSU (Foods and Development), 24, 61-65 (1989)]. Carotenoids such as β-carotene are widely distributed in green plants, so that the plant tissue culture has been examined for the development of a method for producing carotenoids in a large amount which is free from the influence of natural environment [see, for example, Plant Cell Physiol., 12, 525-531 (1971)]. The examination has been also made for detecting a microorganism such as mold, yeast or green algae which is originally high carotenoid productive and for producing carotenoids in a large amount with use of such microorganism (see, for example, The Abstract of Reports in the Annual Meeting of NIPPON HAKKO KOGAKU-KAI of 1988, page 139). However, neither of these methods are successful at present in producing β-carotene at a good productivity which exceeds the synthetic method in commercial production of β-carotene. It would be very useful to obtain a gene group which participates in the biosynthesis of carotenoids, because it will be possible to produce carotenoids in a large amount by introducing a gene group which has been reconstructed to express proper genes in the gene group in a large amount, into an appropriate host such as a plant tissue culture cell, a mold, an yeast or the like which originally produces carotenoids. Such a development in technology has possibilities for finding a method of producing β-carotene superior to the synthetic method and a method of producing useful carotenoids other than β-carotene in a large amount.

Furthermore, the synthesis of carotenoids in a cell or an organ which produces no carotenoid will be possible by obtaining the gene group participating in the biosynthesis of carotenoids, which will add new values to organisms. For example, several reports have recently been made with reference to creating flower colors which cannot be found in nature by using genetic manipulation in flowering plants [see, for example, Nature, 330, 677-678 (1987)]. The color of flowers is developed by pigments such as anthocyanine or carotenoids. Anthocyanine is responsible for flower colors in the spectrum of red-violet-blue, and carotenoids are responsible for flower colors in the spectrum of yellow-orange-red. The gene of the enzyme for synthesizing anthocyanine has been elucidated, and the aforementioned reports for creating a new flower color are those referring to anthocyanine. On the other hand, there are many flowering plants having no bright yellow flower due to no function of synthesizing carotenoids in petal (e.g. petunia, saintpaulia (african violet), cyclamen, Primula malacoides, etc.). If suitable genes having been reconstructed so as to be expressed in petal in a gene group referring to the biosynthesis of carotenoids are introduced into these flowering plants, the flowering plants having yellow flowers will be created successfully.

However, enzymes for synthesizing carotenoids or genes coding for them have been scarcely elucidated at present. The nucleotide sequence of the gene group participating in the biosynthesis of a kind of carotenoids has been elucidated lately only in a photosynthetic bacterium Rhodobacter capsulatus [Mol. Gen. Genet., 216, 254-268 (1989)]. But this bacterium synthesizes the acyclic xanthophyll spheroidene via neurosporene without cyclization and thus cannot synthesize general carotenoids such as lycopene, β-carotene and zeaxanthin.

There are prior arts with reference to yellow pigments or carotenoids of Erwinia species disclosed in J. Bacteriol., 168, 607-612 (1986), J. Bacteriol., 170, 4675-4680 (1988) and J. Gen. Microbiol., 130, 1623-1631 (1984). The first one of these references discloses the cloning of a gene cluster coding for yellow pigment synthesis from Erwinia herbicola Eho 10 ATCC 39368 as a 12.4 kilobase pair (kb) fragment. In this connection, there is no illustration of the nucleotide sequence of the 12.4 kb fragment. The second literature discloses the yellow pigment synthesized by the cloned gene cluster, which is indicated to belong to carotenoids by the analysis of its UV-visible spectrum. The last literature indicates that the gene participating in the production of a yellow pigment is present in a 260 kb large plasmid contained in Erwinia uredovora 20D3 ATTC 19321 from the observation that the yellow pigment is not produced on curing the large plasmid, and further discloses that the pigment belongs to carotenoids from the analysis of its UV-visible spectrum.

However, the chemical structures of carotenoids produced by the Erwinia species or of its metabolic intermediates, enzymes participating in the synthesis of them or the nucleotide sequence of the genes encoding these enzymes remain unknown at present.

### DISCLOSURE OF THE INVENTION

The object of the present invention is to provide DNA sequences which are useful for the synthesis of carotenoids such as lycopene, β-carotene, zeaxanthin or zeaxanthin-diglucoside, that is DNA sequences encoding carotenoid biosynthesis enzymes.

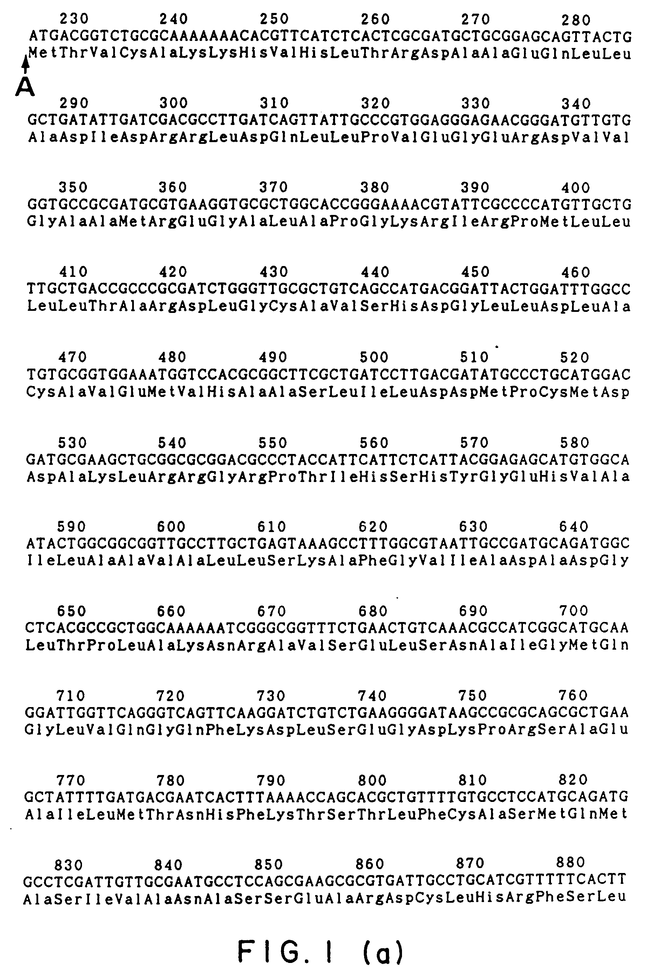

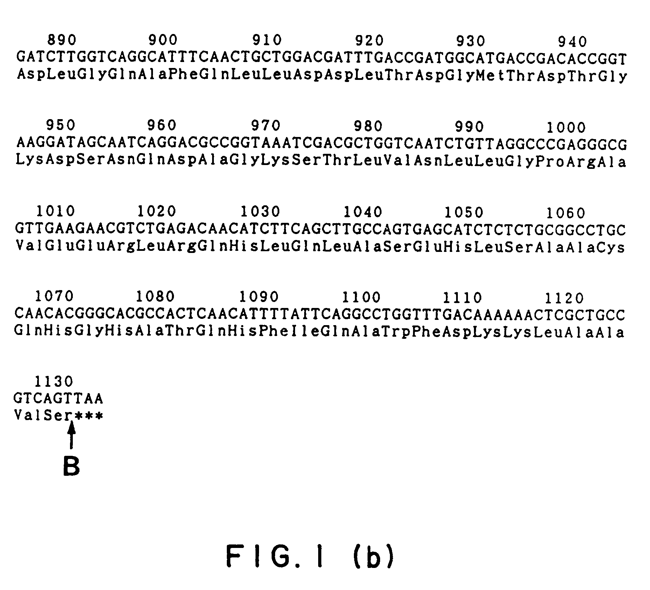

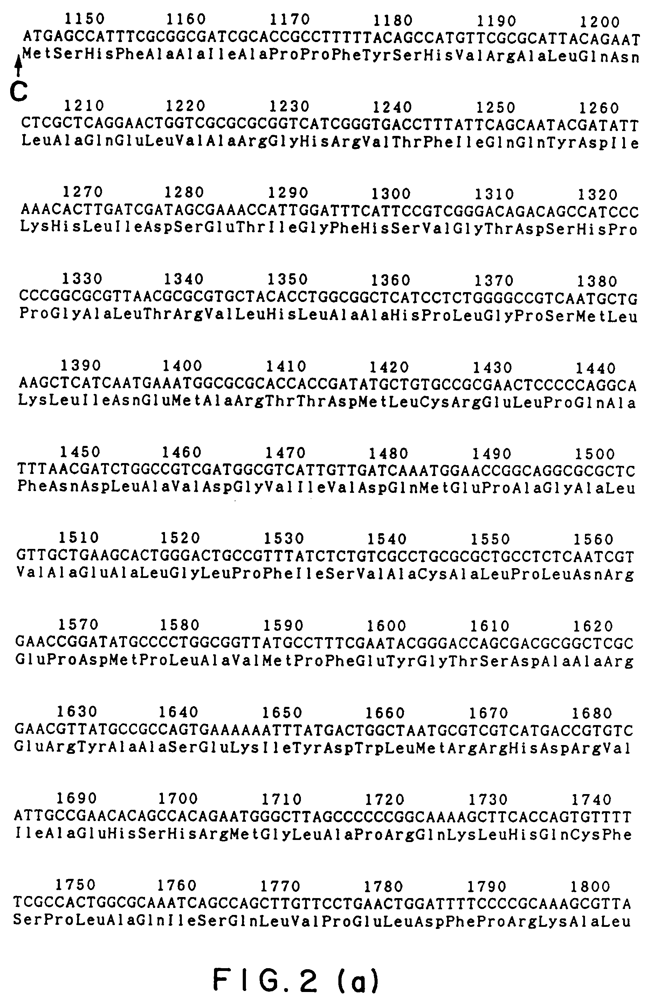

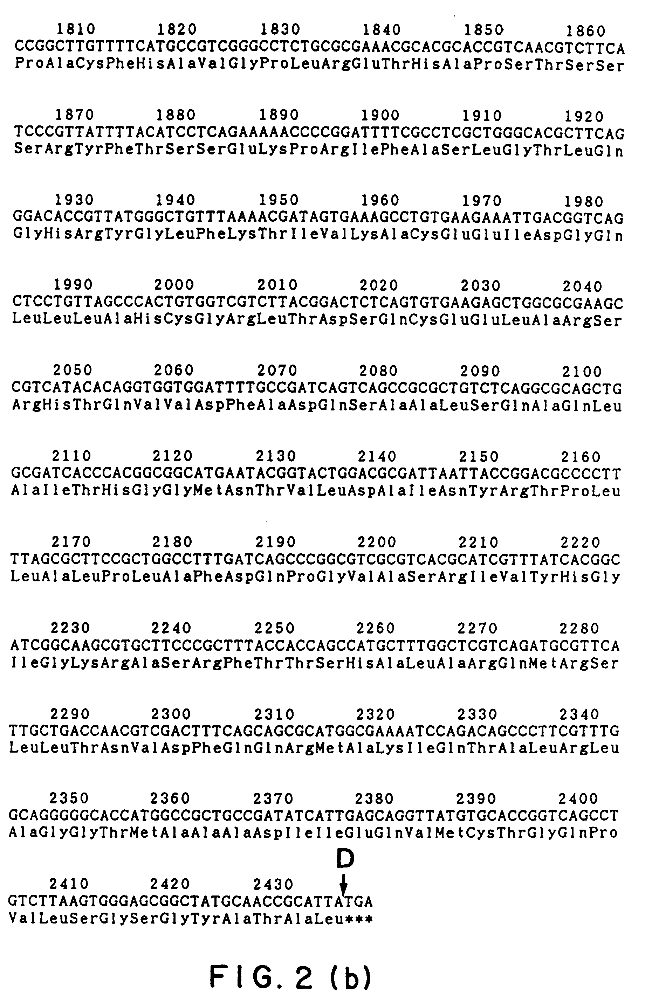

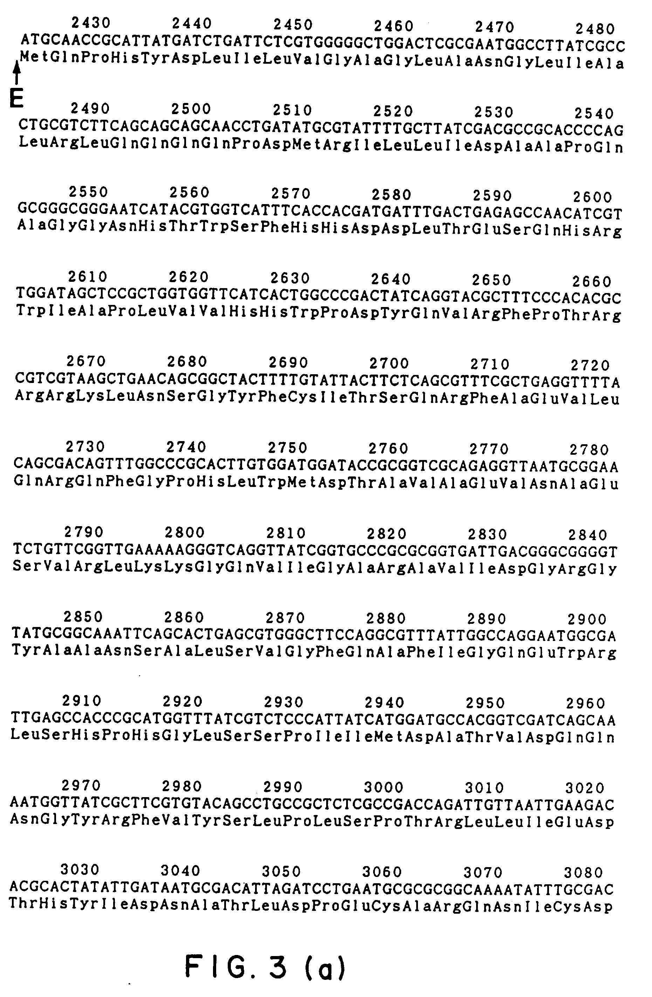

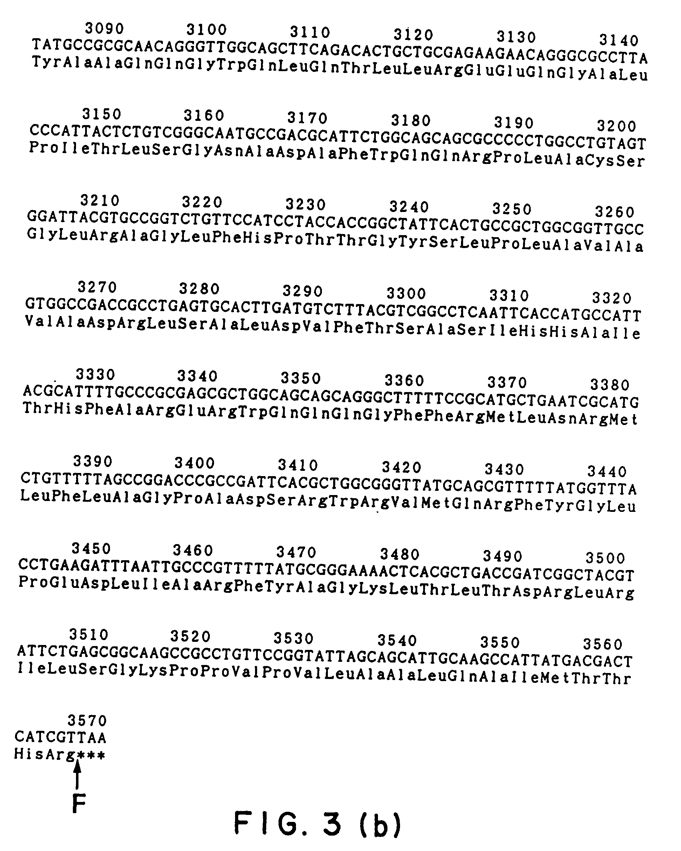

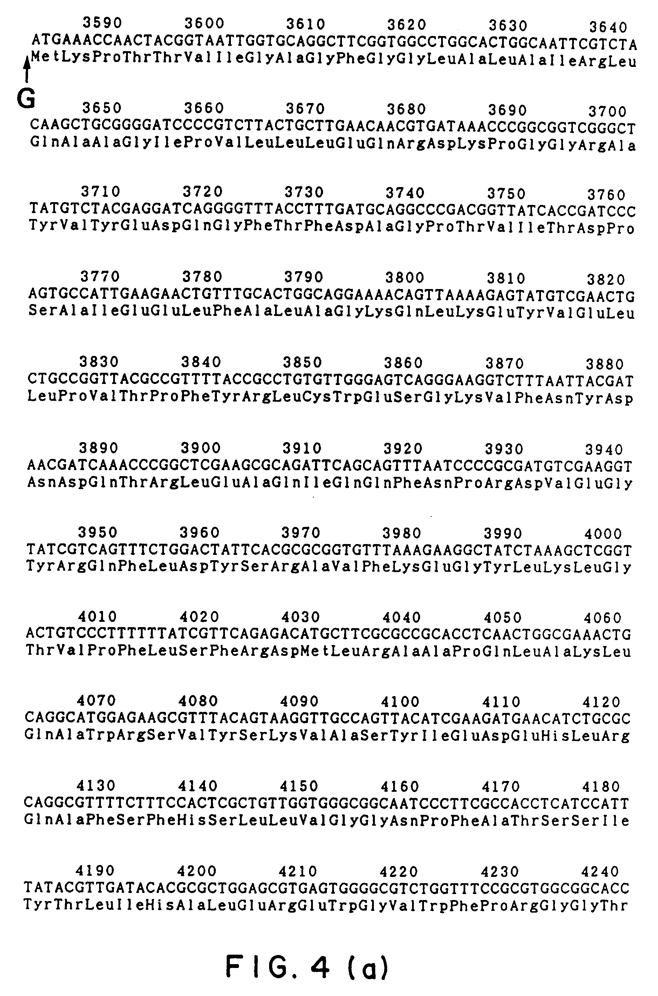

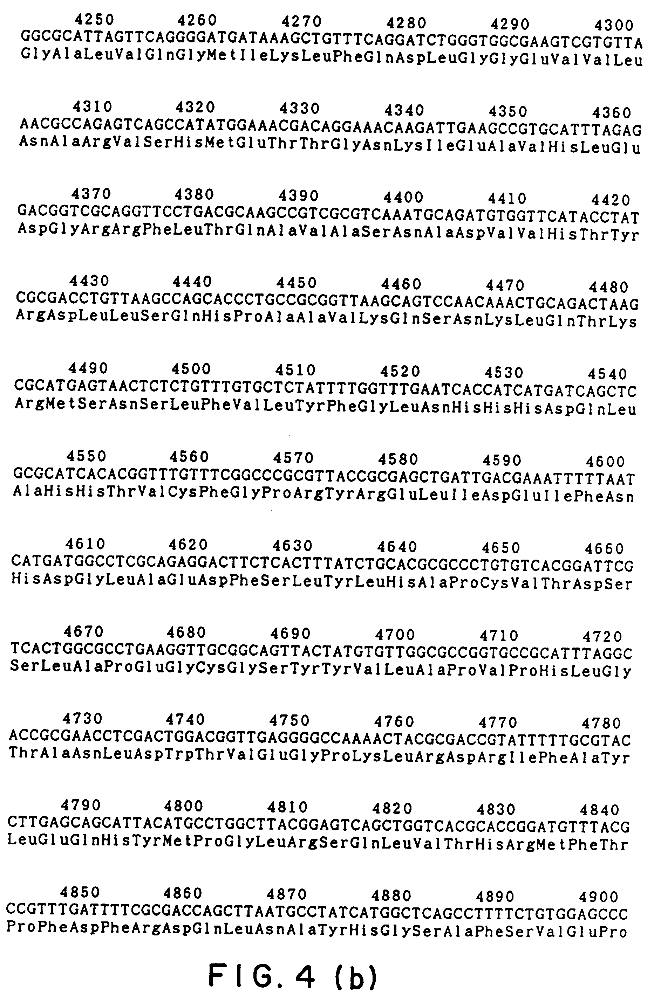

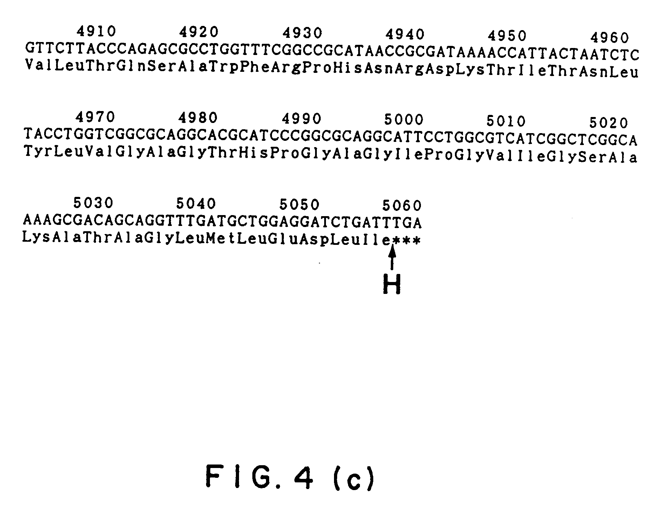

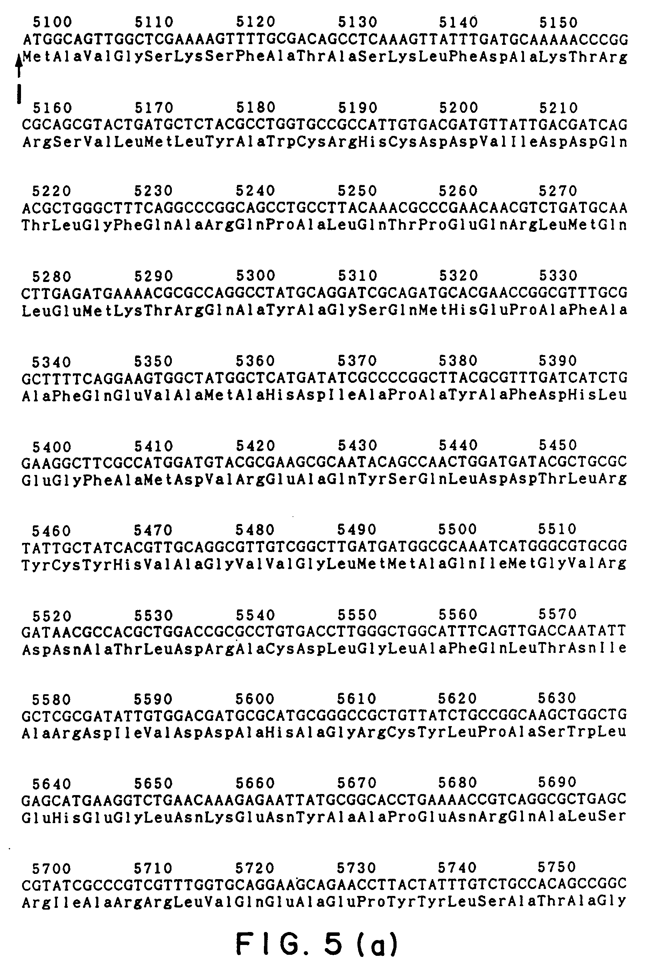

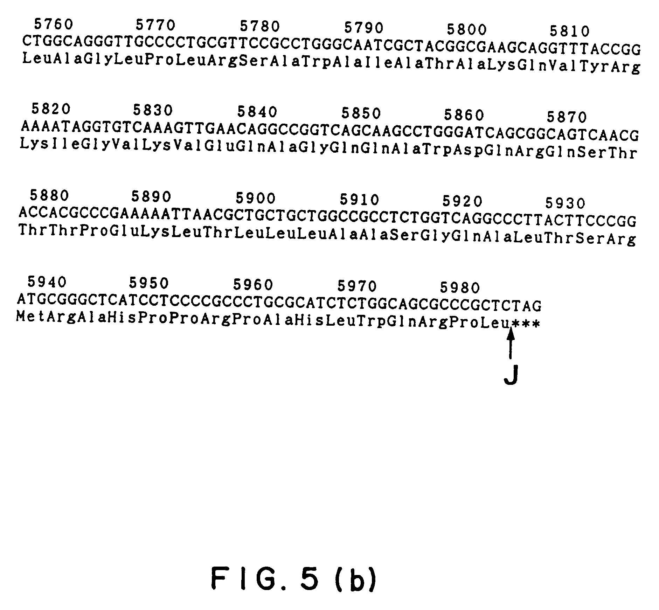

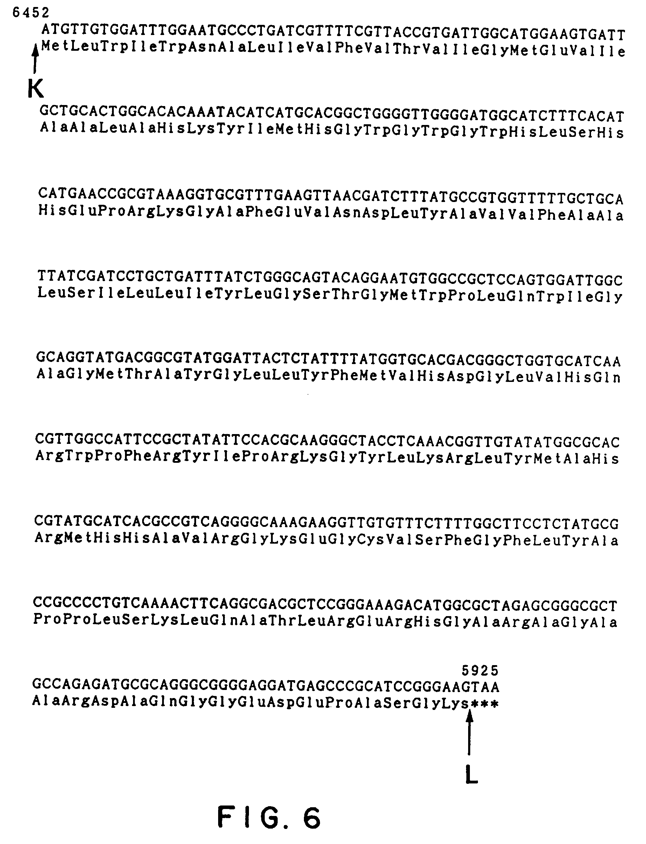

In other words, the DNA sequences useful for the synthesis of carotenoids according to the present invention re the DNA sequences ① - ⑥ described in the following items (1) - (6).
(1) a DNA sequence encoding an enzyme polypeptide which participates in a step before the phytoene stage in carotenoid biosynthesis proceeding via geranylgeranyl pyrophosphate, phytoene and zeaxanthin-diglucoside and whose amino acid sequence corresponds substantially to the amino acid sequence from A to B shown in Figs. 1(a) and (b) (DNA sequence ① );
(2) a DNA sequence encoding a polypeptide which has an enzymatic activity for converting zeaxanthin into zeaxanthin-diglucoside and whose amino acid sequence corresponds substantially to the amino acid sequence from C to D shown in Figs. 2-(a) and (b) (DNA sequence ②);
(3) a DNA sequence encoding a polypeptide which has an enzymatic activity for converting lycopene into β-carotene and whose amino acid sequence corresponds substantially to the amino acid sequence from E to F shown in Figs. 3-(a) and (b) (DNA sequence ③);
(4) a DNA sequence encoding a polypeptide which has an enzymatic activity for converting phytoene into lycopene and whose amino acid sequence corresponds substantially to the amino acid sequence from G to H shown in Figs. 4-(a), (b) and (c) (DNA sequence ④);
(5) a DNA sequence encoding a polypeptide which has an enzymatic activity of converting geranylgeranyl pyrophosphate as a substrate into a next carotenoid compound in the carotenoid biosynthesis proceeding via geranylgeranyl pyrophosphate, phytoene and zeaxanthin-diglucoside and whose amino acid sequence corresponds substantially to the amino acid sequence from I to J shown in Figs. 5-(a) and (b) (DNA sequence ⑤); and
(6) a DNA sequence encoding a polypeptide which has an enzymatic activity for converting β-carotene into zeaxanthin and whose amino acid sequence corresponds substantially to the amino acid sequence from K to L shown in Fig. 6 (DNA sequence ⑥).

Another object of the present invention is to provide processes for producing carotenoid compounds.

More specifically, the present invention also provides a process for producing a carotenoid compound which is related from the group consisting of prephytoene pyrophosphate, phytoene, lycopene, β-carotene, zeaxanthin and zeaxanthin-diglucoside, which comprises transforming a host with at least one of DNA sequences ① - ⑥ described above and culturing the transformant.

### Effect of the Invention

The successful acquirement of the gene group (gene group encoding the biosynthetic enzymes of carotenoids) useful for the synthesis of carotenoids such as lycopene, β-carotene, zeaxanthin, zeaxanthin-diglucoside or the like according to the present invention has made it possible to produce useful carotenoids in large amounts, for example, by creating a plasmid in which the gene(s) can be expressed in a large amount and employing an appropriate plant tissue culture cell, a microorganism or the like transformed with the plasmid. The success in acquiring the gene group useful for the synthesis of carotenoids such as lycopene, β-carotene, zeaxanthin, zeaxanthin-diglucoside or the like according to the present invention has made it possible to synthesize carotenoids in cells or organs which produce no carotenoid by creating a plasmid in which the gene(s) can be expressed in a target cell or organ and transforming a suitable host with this plasmid.

### DETAILED DESCRIPTION OF THE INVENTION

The DNA sequences according to the present invention are the aforementioned DNA sequences ① - ⑥, that is, genes encoding the polypeptides of respective enzymes which participate in the biosynthesis reaction of carotenoids, in particular, for example, such polypeptides in Erwinia uredovora 20D3 ATCC 19321.

A variety of gene groups containing the DNA sequences of a combination of a plurality of sequences among these DNA sequences ① - ⑥ can be expressed in a microorganism, a plant or the like to afford them the biosynthesis ability of carotenoids such as lycopene, β-carotene, zeaxanthin, zeaxanthin-diglucoside or the like. The respective DNA sequences constructing the gene group may be present on a DNA strand or on different DNA strands individually, or optionally, the respective DNA sequences may comprise a plurality of DNA sequences present on a DNA strand and a DNA sequence present on another DNA strand.

The aforementioned gene group encode the polypeptides of a plurality of enzymes participating in the production of carotenoids. A recombinant DNA is created by incorporating the gene group into a proper vector and then introduced into a suitable host to create a transformant, which is cultured to produce mainly in the transformant a plurality of enzymes participating in the formation reaction of carotenoids and to conduct the biosynthesis of carotenoids in the transformant by these enzymes.

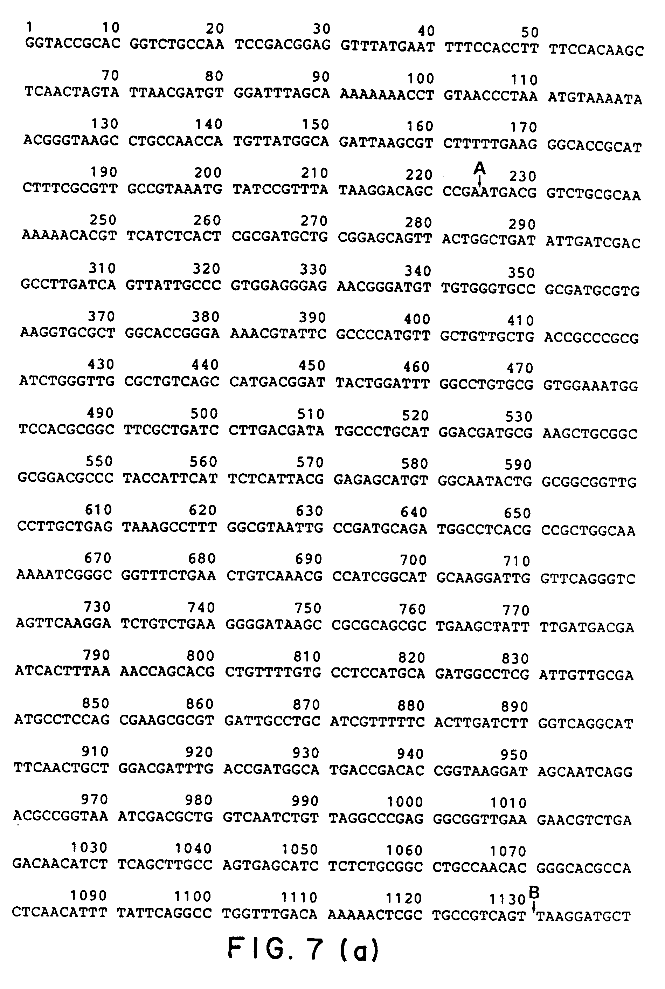

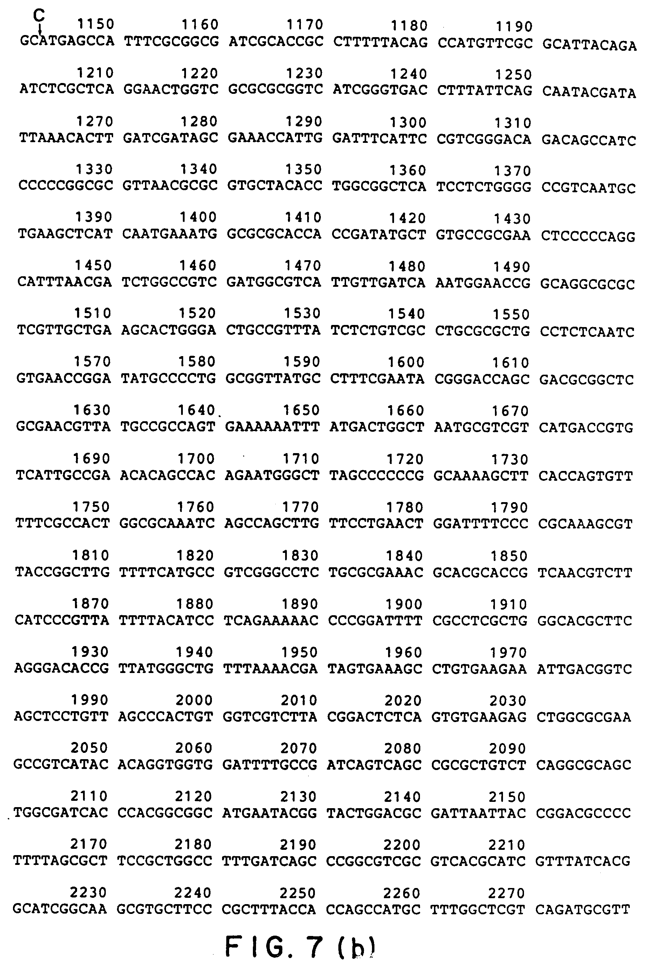

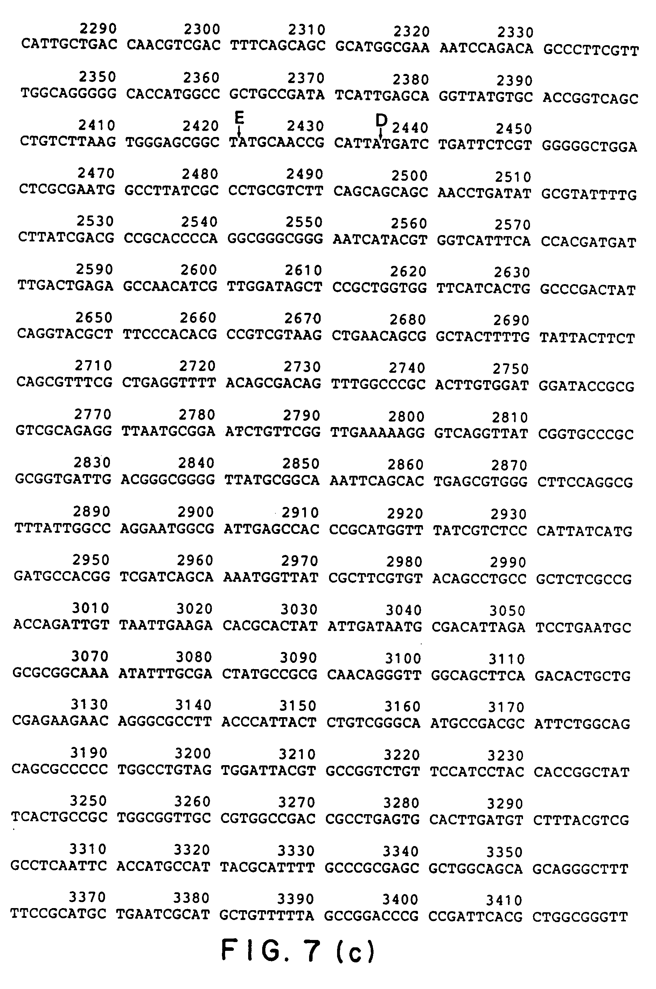

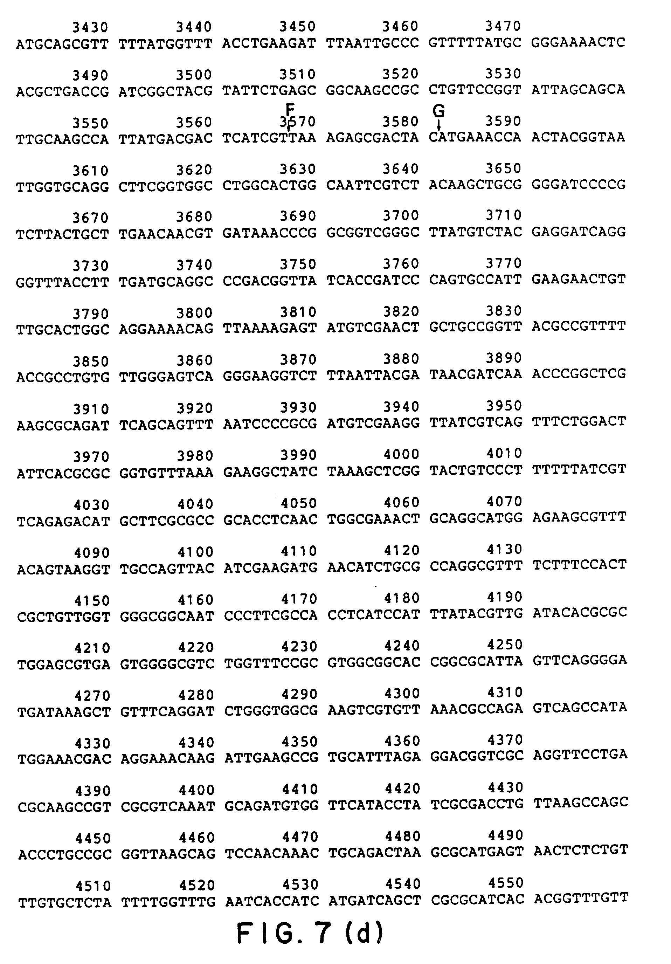

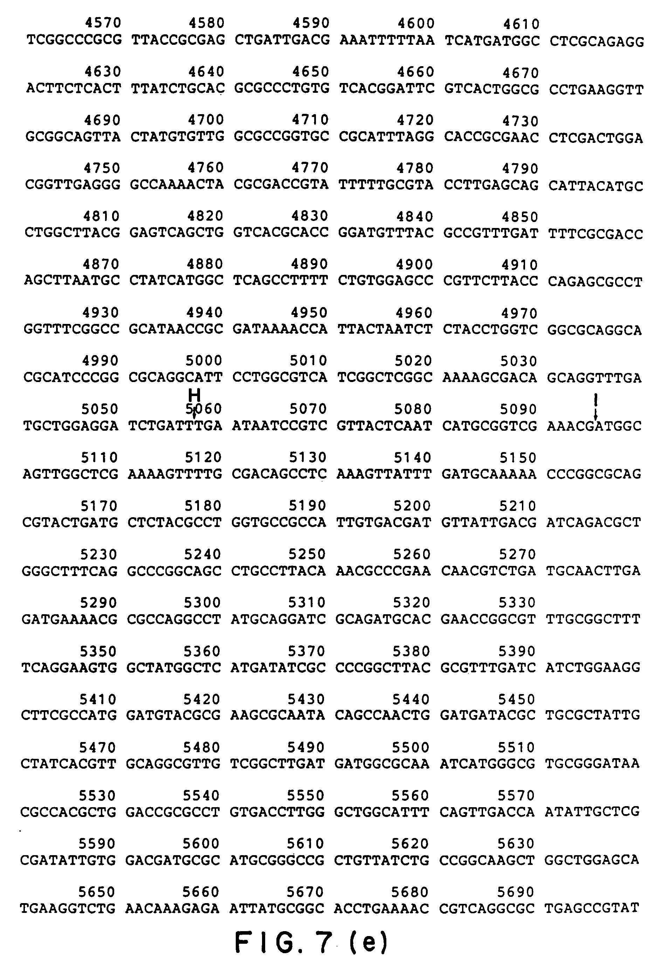

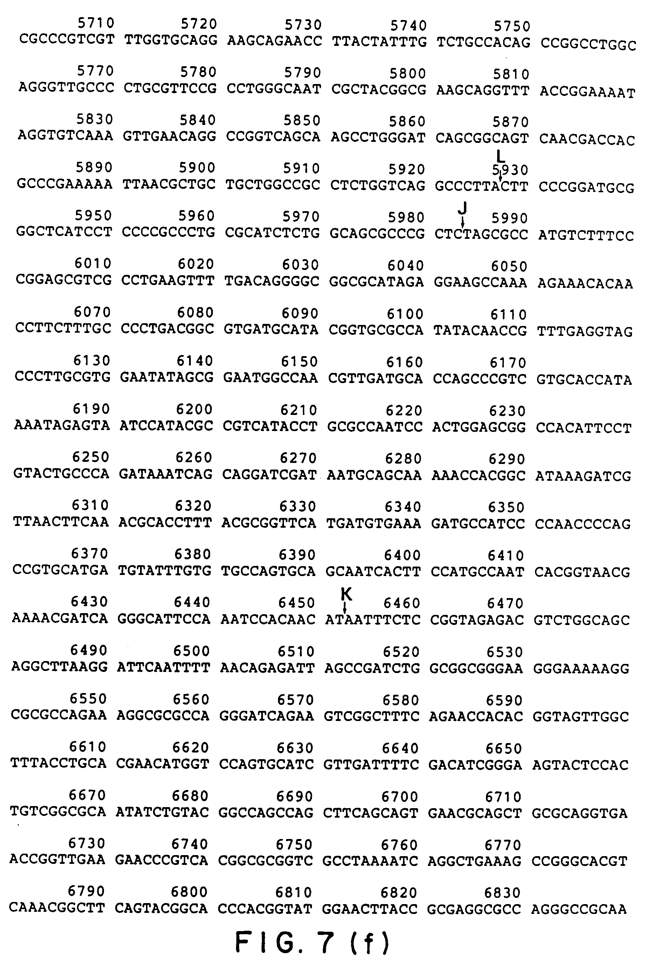

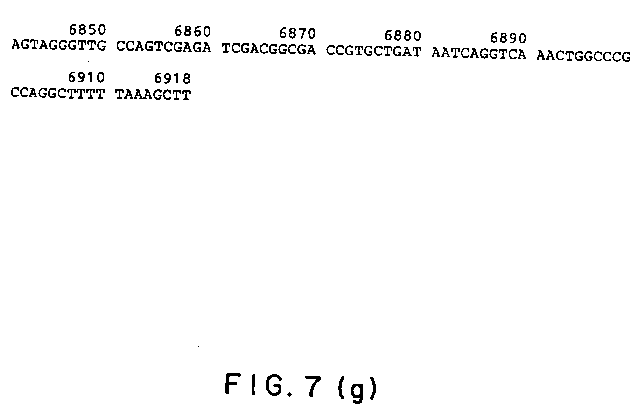

The DNA sequence shown in Fig. 7-(a) to (g), which is an example according to the present invention, is acquired from Erwinia uredovora 20D3 ATCC 19321 and thus exhibits, as illustrated in the experimental example below, no homology in the DNA-DNA hybridization with the DNA strand containing the gene group for synthesizing the yellow pigment of Erwinia herbicola Eho 10 ATCC 39368 (see Related Art described above).

### DNA Sequences encoding the polypeptide of each enzyme

The DNA sequences of the present invention are the DNA sequences ① - ⑥ (or the DNA strands ① - ⑥), respectively. Each of the DNA sequences contains a nucleotide sequence encoding the polypeptide whose amino acid sequence corresponds substantially to such an amino acid sequence as in the aforementioned specific regions in Figs. 1 - 6 (for example, from A to B in Fig. 1). In this connection the term "DNA sequence" means a polydeoxyribonucleic acid sequence having a length. In the present invention, the "DNA sequence" is defined by an amino acid sequence of a polypeptide which is encoded by the DNA sequence and has a definite length as described above, so that each DNA sequence has also a definite length. However, the DNA sequence contains a gene encoding each enzyme and is useful for biotechnological production of the polypeptide, and such biotechnological production cannot be performed by only the DNA sequence having a definite length but can be performed in the state where other DNA sequence with a proper length is linked to the 5′-upstream and/or the 3′-downstream of the DNA sequence. Therefore, the term "DNA sequence" in the present invention includes, in addition to those having a definite length (for example, the length in the region of A - B in the corresponding amino acid sequence of Fig. 1), those in the form of a linear DNA strand or a circular DNA strand containing the DNA sequence having a definite length as a member.

One of the typical forms of each DNA sequence according to the present invention is a form of a plasmid which comprises the DNA sequence as a part of a member or a form in which the plasmid is present in a host such as E. coli. The plasmid as one of the preferable existing forms of each DNA sequence according to the present invention is a conjunction of the DNA sequence according to the present invention as a passenger or a foreign gene, a replicable plasmid vector present stably in a host and a promoter (containing ribosome-binding sites in the case of a procaryote). As the plasmid vector and the promoter, an appropriate combination of those which are well-known can be used.

### Polypeptides encoded by DNA sequences

As mentioned above, the DNA sequences according to the present invention are respectively specified by the amino acid sequences of the polypeptides encoded thereby. Each of these polypeptides is the one having an amino acid sequence which corresponds substantially to an amino acid sequence in a specific region as described above in Figs. 1 - 6 (for example, from A to B in Fig. 1). Here, in the six (A-B, C-D, E-F, G-H, I-J, K-L) polypeptides shown in Figs. 1-6 (i.e. six enzymes participating in the formation of carotenoids), some of the amino acids can be deleted or substituted or some amino acids can be added or inserted, etc., so long as each polypeptide has the aforementioned enzymatic activity in the relationship of a substrate and a converted substance (a product). This is indicated by the expression "whose amino acid sequence corresponds substantially to ..." in the claims. For example, each polypeptide that first amino acid (Met) has been deleted from each polypeptide shown in Figs. 1 - 6 is included in such deleted polypeptides.

The typical polypeptides having enzymatic activities, respectively, in the present invention are those in the specific regions in Figs. 1 - 6 described above, and the amino acid sequences of these polypeptides have not been known.

### Nucleotide sequences of DNA sequences

The DNA sequences encoding the respective enzymes are those having the nucleotide sequences in the aforementioned specific regions in Figs. 1 - 6 (for example, A-B in Fig. 1) or degenerative isomers thereof, or those having the nucleotide sequences corresponding to the aforementioned alteration of the amino acid sequence of respective enzymes or degenerative isomers thereof. The term "degenerative isomer" means DNA sequence which is different only in degenerative codon and can code for the same polypeptide. The preferred embodiments of the DNA sequences according to the present invention are those having at least one stop codon (such as TAA) at the 3′-terminal. The 5′-upstream and/or the 3′-downstream of the DNA sequences according to the present invention may further have a DNA sequence with a certain length as a non-translation region (the initial portion of the 3′-downstream being usually a stop codon such as TAA).

### Gene group used for the synthesis of carotenoids

The gene group (the gene cluster in some case) used for the synthesis of carotenoids comprises a plurality of the aforementioned DNA sequences ① - ⑥, whose typical examples are illustrated in the following (1) - (4). Each gene group encodes a plurality of polypeptides of respective enzymes and these enzymes participate in the production reaction of carotenoids to produce them from their substrates.

### (1) Gene group used for the synthesis of lycopene

The gene group used for the synthesis of lycopene which is a red carotenoid is DNA sequence comprising the aforementioned DNA sequences ①, ④ and ⑤, and such a gene group includes the one in which respective DNA sequences are present on one DNA strand or on different DNA strands separately or the one which is constructed by the combination of the aforementioned ones according to necessities.

In the case that a plurality of DNA sequences are present on one DNA strand, the arrangement order and direction of the aforementioned DNA sequences ①, ④ and ⑤ may be optional provided that the genetic information is capable of expression, that is to say respective genes in a host are in a state of being transcribed and translated appropriately.

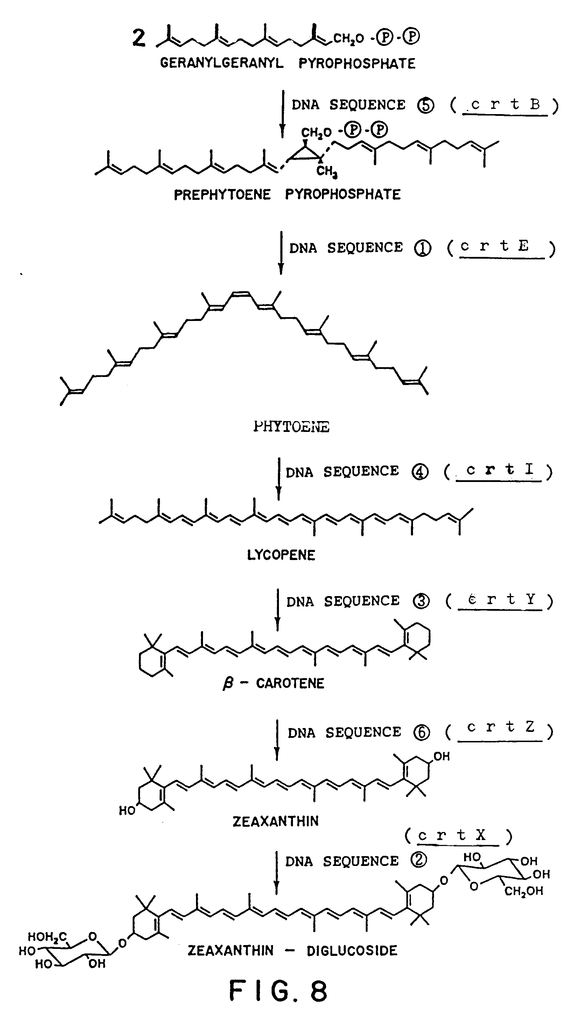

The biosynthetic pathway of lycopene in E. coli is explained as follows: geranylgeranyl pyrophosphate which is a substrate originally present in E. coli is converted into prephytoene pyrophosphate by the enzyme encoded by the DNA sequence ⑤, the prephytoene pyrophosphate is then converted into phytoene by the enzyme encoded by the DNA sequence ①, and the phytoene is further converted into lycopene by the enzyme encoded by the DNA sequence ④ (see Fig. 8).

Lycopene is a carotene whose color is red. Lycopene is a red pigment which is present in a large amount in the fruits of water melon or tomato and has high safety for food. In this connection, the lycopene which was synthesized by the DNA sequences according to the present invention in the experimental example described below had the same stereochemistry as lycopene present in these plants.

One of the typical existing forms of the gene group of the present invention is a form of a plasmid which comprises the respective DNA sequences containing a stop codon as a member or a form in which the plasmid is present in a host such as E. coli. The plasmid which is one of the preferred existing forms of the gene group according to the present invention comprises a gene group as a passenger or a foreign gene, a replicable plasmid vector present stably in a host and a promoter (containing ribosome-binding sites in the case of a procaryote). As the promoter, in procaryotes such as E. coli or Zymomonas species a promoter which is common to respective DNA sequences can be used, or alternatively respective promoters can be used to the respective DNA sequences. In the case of eucaryotes such as yeast or plant, respective promoters are preferably used to respective DNA sequences.

One of the preferred existing forms of the DNA sequences are described above in the explanation of the DNA sequences ① - ⑥.

### (2) Gene group used for the synthesis of β-carotene

The gene group used for the synthesis of β-carotene which is one of yellow-orange carotenoids is a DNA sequence comprising the aforementioned DNA sequences ①, ③, ④ and ⑤. In other words, the gene group used for the synthesis of β-carotene is formed by adding the DNA sequence ③ to a DNA sequence used for the synthesis of lycopene comprising the DNA sequences and ①, ④, and ⑤. The gene group includes the one in which the respective DNA sequences constructing the gene group may be present on one DNA strand or on different DNA strands individually, or the one which is constructed by the combination of the aforementioned ones according to necessities.

In the case that a plurality of DNA sequences are present on one DNA strand, the arrangement order and direction of the aforementioned DNA sequences ①, ③, ④ and ⑤ may be optional provided that the genetic information is capable of expression, that is to say respective genes in a host are in a state of being transcribed and translated appropriately.

The biosynthetic pathway of β-carotene in E. coli is explained as follows: geranylgeranyl pyrophosphate which is a substrate originally present in E. coli is converted into prephytoene pyrophosphate by the enzyme encoded by the DNA sequence ⑤, the prephytoene pyrophosphate is converted into phytoene by the enzyme encoded by the DNA sequence ①, the phytoene is further converted into lycopene by the enzyme encoded by the DNA sequence ④, and the lycopene is further converted into β-carotene by the enzyme encoded by the DNA sequence ③, (see Fig. 8).

β-carotene is a typical carotene whose color is in the spectrum ranging from yellow to orange, and it is an orange pigment which is present in a large amount in the roots of carrot or green leaves of plants and has high safety for food. The utility of β-carotene has already been described in the explanation of related art. In this connection, the β-carotene which was synthesized by the DNA sequence according to the present invention in the experimental example described below had the same stereochemistry as β-carotene present in the roots of carrot or green leaves of plants.

One of the typical existing forms of the gene group and the individual DNA sequences are the same as defined in (1).

### (3) Gene group used for the synthesis of zeaxanthin

The gene group used for the synthesis of zeaxanthin which is one of yellow-orange carotenoids is a DNA sequence comprising the aforementioned DNA sequences ①, ③, ④, ⑤ and ⑥. In other words, the DNA sequence used for the synthesis of zeaxanthin is formed by adding the DNA sequence ⑥ to a DNA sequence used for the synthesis of β-carotene comprising the DNA sequences ①, ③, ④ and ⑤ . The gene group includes the one in which the respective DNA sequences constructing the gene group are present on one DNA strand or on different DNA strands individually, or the one which is constructed by the combination of the aforementioned ones according to necessities.

In the case that a plurality of DNA sequences are present on one DNA strand, the arrangement order and direction of the aforementioned DNA sequences ①, ③, ④, ⑤ and ⑥ may be optional provided that the genetic information is capable of expression, that is to say respective genes in a host are in a state of being transcribed and translated appropriately.

The biosynthetic pathway of zeaxanthin in E. coli is explained as follows: geranylgeranyl pyrophosphate which is a substrate originally present in E. coli is converted into prephytoene pyrophosphate by the enzyme encoded by the DNA sequence ⑤ the prephytoene pyrophosphate is converted into phytoene by the enzyme encoded by the DNA sequence ①, the phytoene is then converted into lycopene by the enzyme encoded by the DNA sequence ④, and the lycopene is further converted into β-carotene by the enzyme encoded by the DNA sequence ③, and finally the β-carotene is converted into zeaxanthin by the enzyme encoded by the DNA sequence ⑥ (see Fig. 8).

Zeaxanthin is a xanthophyll whose color is in the spectrum ranging from yellow to orange, and it is an yellow pigment which is present in the seed of maize and has high safety for food. Zeaxanthin is contained in feeds for hen or colored carp and is an important pigment source for coloring them. In this connection, the zeaxanthin which was synthesized by the DNA sequences according to the present invention in the experimental example described below had the same stereochemistry as zeaxanthin described above.

One of the typical existing forms of the gene group and the individual DNA sequences is the same as defined in (1).

### (4) Gene group used for the synthesis of zeaxanthin-diglucoside

The gene group used for the synthesis of zeaxanthin-diglucoside which is one of yellow-orange carotenoids is a DNA sequence comprising the aforementioned DNA sequences ① - ⑥. In other words, the gene group used for the synthesis of zeaxanthin-diglucoside is formed by adding the DNA sequence ② to a DNA sequence used for the synthesis of zeaxanthin comprising the DNA sequences ①, ③, ④, ⑤ and ⑥. The gene group includes the one in which the respective DNA sequences constructing the gene group are present on one DNA strand or on different DNA strands individually, or the one which is constructed by the combination of the aforementioned ones according to necessities.

In the case that a plurality of DNA sequences are present on one DNA strand, the arrangement order and direction of the aforementioned DNA sequences ① - ⑥ may be optional provided that the genetic information is capable of expression, that is to say respective genes in a host are in a state of being transcribed and translated appropriately.

One of the typical existing forms of the gene group and the individual DNA sequences is the same as defined in (1).

The biosynthetic pathway of zeaxanthin-diglucoside in E. coli is explained as follows: geranylgeranyl pyrophosphate which is a substrate originally present in E. coli is converted into prephytoene pyrophosphate by the enzyme encoded by the DNA sequence ⑤, the prephytoene pyrophosphate is converted into phytoene by the enzyme encoded by the DNA sequence ①, the phytoene is then converted into lycopene by the enzyme encoded by the DNA sequence ④, and the lycopene is further converted into β-carotene by the enzyme encoded by the DNA sequence ③, the β-carotene is then converted into zeaxanthin by the enzyme encoded by the DNA sequence ⑥, and the zeaxanthin is finally converted into zeaxanthin-diglucoside by the enzyme encoded by the DNA sequence ② (see Fig. 8).

Zeaxanthin-diglucoside is a carotenoid glycoside having a high water solubility and a pigment which is soluble sufficiently in water at room temperature and exhibits clear yellow. Carotenoid pigments are generally hydrophobic and thus limited on their use as natural coloring materials in foods or the like. Therefore, zeaxanthin-diglucoside settles this defect. Zeaxanthin-diglucoside is isolated from edible plant saffron, Croccus sativus (Pure & Appl. Chem., 47, 121-128 (1976)), so that it is thought that its safety for food has been confirmed. Therefore, zeaxanthin-diglucoside is desirable as a yellow natural coloring material of foods or the like. In this connection, there has been heretofore no reports with reference to the isolation of zeaxanthin-diglucoside from microorganisms.

If carotenoid pigments such as lycopene, β-carotene, zeaxanthin and zeaxanthin-diglucoside are intended to be produced, the aforementioned DNA sequences ①, ④ and ⑤, the DNA sequences ①, ③, ④ and ⑤, the DNA sequences ①, ③, ④, ⑤ and ⑥, and the DNA sequences ① - ⑥ are required, respectively, on using E. coli as the host. However, when a host other than E. coli, particularly the one which is capable of producing carotenoids is used, it has a high possibility of containing also carotenoid precursors at further downstream in the biosynthesis, so that all of the aforementioned DNA sequences ①, ④ and ⑤ (for the production of lycopene), all of the DNA sequences ①, ③, ④ and ⑤ (for the production of β-carotene), all of the DNA sequences ①, ③, ④, ⑤ and ⑥ (for the production of zeaxanthin), or all of the DNA sequences ① - ⑥ (for the production of zeaxanthin-diglucoside) are not always required.

That is to say, only the DNA sequence(s) participating in the formation of an aimed carotenoid pigment from a carotenoid precursor present at the furthest downstream in the host may also be used in this case. Thus, when lycopene is intended to be produced as an aimed carotenoid in a host in which phytoene is preliminarily present, it is also possible to use only the DNA sequence ④ among the DNA sequences ①, ④ and ⑤.

It is also possible to make a host to produce, as the aimed carotenoid pigment relating compound, prephytoene pyrophosphate from geranylgeranyl pyrophosphate by using only the DNA sequence ⑤ of the present invention, or phytoene by using the DNA sequences ① and ⑤ of the present invention or, if the host contains prephytoene pyrophosphate, by using only the DNA sequence ① .

### Acquirement of DNA sequences

A method for acquiring the DNA sequences ① - ⑥ which contain the nucleotide sequences coding for the amino acid sequences of the respective enzymes is the chemical synthesis of at least a part of their strand by the method of polynucleotide synthesis. However, if it is taken into consideration that a number of amino acids are bonded, it would be more preferable than the chemical synthesis to acquire the DNA sequences from the DNA library of Erwinia uredovora 20D3 ATTC 19321 according to a conventional method in the field of genetic engineering, for example, the hybridization method with a suitable probe.

The individual DNA sequences or the DNA sequence comprising all of these sequences are thus obtained.

### Transformant

The aforementioned gene group comprising a plurality of the DNA sequences ① - ⑥ can be constituted by using the DNA sequences obtained as described above. The DNA sequence thus obtained contains genetic informations for making an enzyme participating in the formation of carotenoids, so that it can be introduced into an appropriate host by the biotechnological method to form a transformant and to produce an enzyme and in its turn a carotenoid pigment or a carotenoid pigment relating compound.

### (1) Host

Plants and a variety of microorganisms, as far as a suitable host-vector system is present, can be the target of transformation by a vector comprising the aforementioned DNA sequences. However, the host is required to contain geranylgeranyl pyrophosphate which is a substrate compound of an enzyme for starting the carotenoid synthesis with use of the DNA sequences of the present invention, or a compound further downstream from it.

It is known that geranylgeranyl pyrophosphate is synthesized by dimethylallyltransferase which is a common enzyme at the initial stage of the biosynthesis of not only carotenoids but also sterols or terpenes [J. Biochem., 72, 1101-1108 (1972)]. Accordingly, if a cell which cannot synthesize carotenoids can synthesize sterols or terpenes, it probably contains geranylgeranyl pyrophosphate. It is believed that a cell contains at least one of sterols or perpenes.

Therefore, it is believed theoretically that almost all hosts are capable of synthesizing carotenoids by using the DNA sequences of the present invention as far as a suitable host-vector system is present.

As the hosts in which the host-vector system is present, there are mentioned plants such as Nicotiana tabacum, Petunia hybrida and the like, microorganism such as bacteria, for example Escherichia coli, Zymomonas mobilis and the like, and yeasts, for example Saccharomyces cerevisiae and the like.

### (2) Transformation

It is confirmed for the first time by the present invention that the genetic informations present on the DNA sequences of the present invention has been expressed in microorganisms. However, the procedures or the methods for making the transformants (and the production of enzymes or in its turn carotenoid pigments or carotenoid pigment relating compounds by the transformants) are per se conventional in the fields of molecular biology, cell biology or genetic manipulation, and thus the procedures other than described below may be performed in accordance with these conventional techniques.

In order to express the gene of the DNA sequences according to the present invention in a host, it is necessary to insert the gene into a vector for introducing it into the host. As the vector used in this stage, there is used all of various known vectors such as pBI121 or the like for plants (Nicotiana tabacum, Petunia hybrida); pUC19, pACYC184 or the like for E. coli; pZA22 or the like for Zymomonas mobilis (see Japanese Patent Laid-Open Publication No. 228278/87); and YEp13 or the like for yeast.

On the other hand, it is necessary to transcribe the DNA sequence of the present invention onto mRNA in order to express the gene of the DNA sequence in the host. For this purpose, a promoter as a signal for the transcription may be integrated into the 5′-upstream region from the DNA sequence of the present invention. A variety of promoters such as CaMV35S, NOS, TR1′, TR2′ (for plants); lac, Tc^{r}, CAT, trp (for E. coli); Tc^{r}, CAT (for Zymomonas mobilis); ADH1, GAL7, PGK, TRP1 (for yeast) and the like are known as for the promoters, and either of these promoters can be used in the present invention.

In the case of procaryote, it is necessary to place ribosome-binding site (SD sequence in E. coli) several base-upstream from the initiation codon (ATG).

In this connection, while the aforementioned manipulation is necessary for producing the enzyme protein, one or more of amino acids may be inserted into or added to the polypeptide which is illustrated in the specific ranges of Figs. 1 - 6 (e.g. the polypeptide A - B illustrated in Fig. 1), one or more of amino acids may be deleted, or replaced, as described above.

The transformation of the host with the plasmid thus obtained can be conducted optionally by an appropriate method which is conventionally used in the fields of genetic manipulation or cell biology. As for the general matters, there can be referred to appropriate publications or reviews; for example as for the transformation of microorganisms, T. Maniatis, E. F. Fritsch and J. Sambrook: "Molecular Cloning A Laboratory Manual", Cold Spring Harbor Laboratory (1982).

The transformant is the same as the host used, in its genotype, phenotype or bacteriological properties but for the new trait derived from the genetic information introduced by the DNA sequence of the present invention (that is, the production of an enzyme participating in the carotenoid formation and the synthesis of carotenoids or the like by the enzyme), the trait derived from the vector used and the deletion of the trait corresponding to the deletion of a part of the genetic information of the vector which might be caused on the recombination of genes. Escherichia coli JM109 (pCAR1) which is an example of the transformant according to the present invention is deposited as FERM BP-2377.

### Expression of genetic information/production of carotenoids

The clone of the transformant obtained as described above produces mainly in the transformant an enzyme participating in the carotenoid formation, and a variety of carotenoids or carotenoid pigment relating compounds are synthesized by the enzyme.

Culture or the culturing condition of the transformant is essentially the same as those for the host used.

Carotenoids can be recovered by the methods, for example, illustrated in Experimental Examples 3 and 4 below.

Furthermore, each enzyme protein coded by each DNA sequence of the present invention is produced mainly in the cell in the case of the transformation of E. coli, and it can be recovered by an appropriate method.

### BRIEF DESCRIPTION OF THE DRAWINGS

Figs. 1 - 6 illustrate the nucleotide sequences in the DNA sequences ① - ⑥ in coding regions, and the amino acid sequences of proteins to be encoded, respectively,
Fig. 7 illustrates the KpnI-HindIII fragtment which was acquired from Erwinia uredovora 20D3 ATCC 19321 and relates to the biosynthesis of carotenoids, that is the complete nucleotide sequence of the 6918 bp DNA sequence containing the DNA sequences in Figs. 1 - 6, and
Fig. 8 illustrates the function of the polypeptides encoded by the aforementioned DNA sequences ① - ⑥.

### Experiments

All of strains used in the following experiments are deposited in ATCC or other deposition organizations and are freely available.

### Experimental Example 1: Cloning of a gene cluster participating in the biosynthesis of a yellow pigment (referred to hereinafter as yellow pigment-synthesizing gene cluster)

### (1) Preparation of total DNA

Total DNA was prepared from the cells of Erwinia uredovora 20D3 ATCC 19321 which had been proliferated until the early-stationary phase in 100 ml of LB medium (1% tryptone, 0.5% yeast extract, 1% NaCl). Penicillin G (manufactured by Meiji Seika) was added to the culture medium so that it has a concentration of 50 units/ml in the medium before 1 hour of the harvest of the cells. After harvesting the cells by centrifugation, this was washed with the TES buffer (20 mM tris, 10 mM EDTA, 0.1 M NaCl, pH 8), heat treated at 68°C for 15 minutes and suspended in Solution I (50 mM glucose, 25 mM Tris, 10 mM EDTA, pH 8) containing 5 mg/ml of lysozyme (manufactured by Seikagaku Kogyo) and 100 »g/ml of RNase A (manufactured by Sigma). The suspension was incubated at 37°C for a period of 30 minutes - 1 hour, and pronase E (manufactured by Kaken Seiyaku) was added so that it had a concentration of 250 »g/ml before incubation at 37°C for 10 minutes. Sodium N-lauroylsarcosine (manufactured by Nacalai tesque) was added so as it had the final concentration of 1%, and the mixture was agitated before incubation at 37°C for several hours. Extraction was conducted several times with phenol/chloroform. While ethanol in volume of 2 equivalents was slowly added, the resulting total DNA was wound around a glass stick, rinsed with 70% ethanol and dissolved in 2 ml of TE buffer (10 mM Tris, 1 mM EDTA, pH 8) to give the total DNA preparation.

### (2) Construction of an Escherichia coli cosmid library and acquirement of E. coli transformants producing yellow pigments

Incubation was conducted with 1 unit of restriction enzyme Sau3AI per 50 »l of the total DNA preparation at 37°C for 30 minutes before the inactivation treatment of the restriction enzyme at 68°C for 10 minutes. Many fragments partially digested with Sau3AI were obtained in the neighbourhood of 40 kb under this condition. After the ethanol precipitation of this reaction solution, this half portion was mixed with 2.5 »g of cosmid pJB8 which had been digested with BamHI and treated with alkaline phosphatase and 0.2 »g of a pJB8 SalI-BamHI right arm fragment (smaller fragment) which had been recovered from a gel, and 40 »l of the total amount was subjected to ligation reaction with T4 DNA ligase at 12°C for 2 days. In this connection, the cosmid pJB8 had been previously purchased from Amersham. Restriction enzymes and enzymes used for genetic manipulation were purchased from Boehringer-Mannheim, Takara Shuzo or Wako Pure Chemical Industries. This DNA in which the ligation reaction had been thus performed was used for in vitro packaging with a Gigapack Gold (manufactured by Stratagene, marketed from Funakoshi) to give a large amount of phage particles sufficient for construction of a cosmid library. The phage particles were infected with Escherichia coli DH1 (ATCC 33849). After the cells of E. coli DH1 infected were diluted so as to be 100 colonies per plate, they were plated on a LB plate, cultured at 37°C overnight and further at 30°C for 6 hours or more. As a result, E. coli transformants producing yellow pigments appeared in a proportion of one colony per about 1,100 colonies. These E. coli transformants producing yellow pigments contained plasmids in which 33 - 47 kb Sau3AI partial digestion fragments were inserted into the pJB8.

### (3) Location of a yellow pigment-synthesizing gene cluster

A yellow pigment-synthesizing gene cluster was inserted into the pJB8 as the 33 - 47 kb Sau3AI partial digestion fragments. One of these fragments was further subjected to partial digestion with Sau3AI, ligated to the BamHI site of the E. coli vector pUC19 (purchased from Takara Shuzo), and used to transform Escherichia coli JM109 (manufactured by Takara Shuzo). To locate the yellow pigment-synthesizing gene cluster, plasmid DNA's were prepared from 50 E. coli transformants producing yellow pigments which appeared in the LB plate containing ampicillin, and analyzed by agarose gel electrophoresis. As a result, it was found that the smallest inserted fragment was of 8.2 kb. The plasmid containing this 8.2 kb fragment was named as pCAR1 and E. coli JM109 harboring this plasmid was named as Escherichia coli JM109 (pCAR1). This strain produced the same yellow pigments as those of E. uredovora. The 8.2 kb fragment contained a KpnI site in the neighbourhood of the terminal at the lac promoter side and a HindIII site in the neighbourhood at the opposite side. After the 8.2 kb fragment was subjected to double digestion with KpnI/HindIII (HindIII was partially digested; the 8.2 kb fragment had two HindIII sites), the KpnI-HindIII fragment (6.9 kb) was recovered from a gel and ligated to the KpnI-HindIII site of pUC18 (this hybrid plasmid was named as pCAR15). Upon the transformation of E. coli JM109, the E. coli transformant exhibited yellow and produced the same yellow pigments as those of E. uredovora. Accordingly, it was found out that the genes required for the yellow pigment production was located on the KpnI-HindIII fragment (6.9 kb). That is to say, the fragment carrying the yellow pigment-synthesizing genes was capable of being reduced to a 6.9 kb in size.

### Experimental Example 2: Analysis of the yellow pigment-synthesizing gene cluster

### (1) Determination of the nucleotide sequence of the yellow pigment-synthesizing gene cluster

The complete nucleotide sequence of the 6.9 kb KpnI-HindIII fragment was determined by the kilo-sequence method using Deletion kit for kilo-sequence (manufactured by Takara Shuzo) and the dideoxy method according to Proc. Natl. Acad. Sci. USA, 74 5463-5467 (1977). As a result, it was found that the KpnI-HindIII fragment containing the yellow pigment-synthesizing genes (DNA strand) was 6918 base pairs (bp) in length and its GC content was 54%. The complete nucleotide sequence was shown in Fig. 7 (a) - (g). The KpnI site is represented by the base number 1.

### (2) Elucidation of yellow pigment-synthesizing gene cluster

The HindIII side of the 6918 bp fragment (DNA strand) containing the yellow pigment-synthesizing genes (right terminal side in Fig. 7) was deleted with Deletion kit for kilo-sequence. A hybrid plasmid (designated pCAR25) was constructed by inserting a 1 - 6503 fragment, which was obtained by deletion from the HindIII site to nucleotide position 6504, into pUC19. E. coli JM109 harboring pCAR25 [referred to hereinafter as E. coli (pCAR25)] exhibited yellow and produced the same yellow pigments as those of E. uredovora. Therefore, it was thought that the region from the base number 6504 to 6918 in Fig. 7 was not required for yellow pigment production. The nucleotide sequence in the region from the base number 1 to 6503 in the 6918 bp DNA sequence containing the yellow pigment-synthesizing genes was analyzed. As a result, it was found that there were six open reading frames (ORFs). That is to say, there were an ORF coding for a polypeptide with a molecular weight of 32,583 from the base number 225 to 1130 (referred to as ORF1, which corresponds to A - B in Figs. 1 and 7), an ORF coding for a polypeptide with a molecular weight of 47,241 from the base number 1143 - 2435 (referred to as ORF2, which corresponds to C - D in Figs. 2 and 7), an ORF coding for a polypeptide with a molecular weight of 43,047 from the base number 2422 to 3567 (referred to as ORF3, which corresponds to E - F in Figs. 3 and 7), an ORF coding for a polypeptide with a molecular weight of 55,007 from the base number 3582 to 5057 (referred to as ORF4, which corresponds to G - H in Figs. 4 and 7), an ORF coding for a polypeptide with a molecular weight of 33,050 from the base number 5096 to 5983 (referred to as ORF5, which corresponds to I - J in Figs. 5 and 7), and an ORF coding for a polypeptide with a molecular weight of 19,816 from the base number 6452 to 5928 (referred to as ORF6, which corresponds to K - L in Figs. 6 and 7. Only this ORF6 has the opposite orientation with the others). In this connection, each ORF contained at positions several base-upstream from its initiation codon the SD (Shine-Dalgarno) sequence which is homologous with the 3'-region of 16S ribosomal RNA of E. coli. Thus, it was thought that polypeptides were in fact synthesized in E. coli by these six ORFs. This was confirmed by the following in vitro transcription-translation experiment.

That is to say, the in vitro transcription-translation analysis was carried out with DNA in which the plasmid pCAR25 containing ORF1 - ORF6 had been digested with ScaI and with DNAs in which respective fragments containing respective ORFs (containing the SD sequence) of ORF1 - ORF6 had been digested with appropriate restriction enzymes, isolated, inserted into pUC19 or pUC18 so that it was subjected to transcriptional read-through from a lac promoter, and then digested with ScaI. In this experiment, a Prokaryotic DNA-directed translation kit manufactured by Amersham was used. As a result, it was confirmed that the bands of polypeptides corresponding to the aforementioned respective ORFs were detected as the transcription-translation products.

Moreover, all of six ORFs were necessary for production of the same yellow pigments as those of E. uredovora as described below (Experimental Examples 3, 4 and 5). From these results, ORF1, ORF2, ORF3, ORF4, ORF5 and ORF6 were designated as crtE, crtX, crtY, crtI, crtB and crtZ genes, respectively.

The base numbers in Figs. 1 - 6 were represented on the basis of the KpnI site in Fig. 7 as the base number 1 and correspond to each other. The marks A - L in Figs. 1 - 6 correspond to the marks A - L in Fig. 7. The DNA sequence from K to L in Fig. 6 was that of the complementary strand of the DNA sequence from K to L in Fig. 7. That is to say, the DNA sequence illustrated in Fig. 6 has the opposite orientation in transcription with the DNA sequences in Figs. 1 - 5 in the original DNA sequence (Fig. 7).

### (3) Analysis of homology by the DNA-DNA hybridization method

Total DNA of Erwinia herbicola Eho 10 ATCC 39368 was prepared in the same manner as in Experimental Example 1 (1). A 7.6 kb fragment containing the DNA sequence in Fig. 7 was cut out from the hybrid plasmid pCAR1 by KpnI digestion and labeled with DNA labeling & detection kit nonradioactive (manufactured by Boehringer-Mannheim) according to the DIG-ELISA method to give probe DNA. The homology of total DNAs (intact or KpnI digested) of E. herbicola Eho 10 ATCC 39368 and E. uredovora 20D3 ATCC 19321 with this probe DNA was analyzed by the DNA-DNA hybridization method with the aforementioned DNA labeling & detection kit nonradioactive. As a result, the probe DNA was hybridized strongly with total DNA of the latter E. uredovora 20D3 ATCC 19321, but not at all with total DNA of the former E. herbicola Eho 10 ATCC 39368. Also, the restriction map deduced from the DNA sequence in Fig. 7 was quite different from that reported in J. Bacteriol., 168, 607-612 (1986). It was concluded from the above described results that the DNA sequence in Fig. 7, that is, the DNA sequences useful for the synthesis of carotenoids according to the present invention exhibits no homology with the DNA sequence containing the yellow pigment-synthesizing genes of E. herbicola Eho 10 ATCC 39368.

### Experimental Example 3: Analysis of yellow pigments

E. coli (pCAR25) produced the same yellow pigments as those of E. uredovora 20D3 ATCC 19321 and E. herbicola Eho 10 ATCC 39368, and its yield was 5 times higher than those of the former and 6 times higher than those of the latter (per dry weight). The cells harvested from 8 liters of 2 × YT medium (1.6% tryptone, 1% yeast extract, 0.5% NaCl) were extracted once with 1.2 liter of methanol. The methanol extract was evaporated to dryness, dissolved in methanol, and subjected to thin layer chromatography (TLC) with silica gel 60 (Merck) (developed with chloroform : methanol = 4:1). The yellow pigments were separated into 3 spots having Rf values of 0.93, 0.62 and 0.30 by TLC. The yellow (to orange) pigment at the Rf value of 0.30 which was the strongest spot was scraped up from the TLC plate, extracted with a small amount of methanol, loaded on a Sephadex LH-20 column for chromatography [30 cm × 3.0 cm (⌀)] and developed and eluted with methanol to give 4 mg of a pure product. The yellow (to orange) pigment obtained was sparingly soluble in organic solvents other than methanol and easily soluble in water, so that it was suggested that it might be a carotenoid glycoside. Such suggestion was also supported from a molecular weight of 892 by FD-MS spectrum (the mass of this pigment was larger than that of zeaxanthin (described hereinafter) by the mass of two glucose). When this substance was hydrolyzed with 1N HCl at 100°C for 10 minutes, zeaxanthin was obtained. Then, acetylation was conducted according to the usual method. That is, the substance was dissolved in 10 ml of pyridine, large excess of acetic anhydride was added, and the mixture was stirred at room temperature and left standing overnight. After the completion of reaction, water was added to the mixture and chloroform extraction was carried out. The chloroform extract was concentrated and loaded on a silica gel column [30 cm × 3.0 cm (⌀)] for chromatography to develop and elute with chloroform. Measurement of ¹H-NMR gave the spectrum identical with the tetraacetyl derivative of zeaxanthin-β-diglucoside [Helvetica Chimica Acta, 57, 1641-1651 (1974)], so that the substance was identified as zeaxanthin-β-diglucoside (its structure being illustrated below).

The yield was 1.1 mg/g dry weight. The substance had a solubility of at least 2 mg in 100 ml of water and methanol, and water was superior to methanol in solubility of the substance. The substance had low solubilities in chloroform and acetone, and its solubilities were 0.5 mg in 100 ml of these solvents.

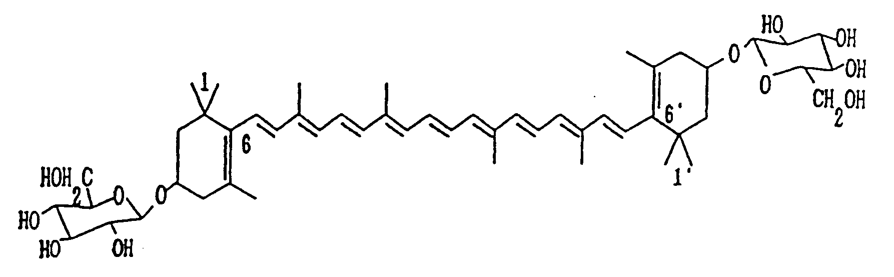

### Experimental Example 4: Analysis of the metabolic intermediates of carotenoids

### (1) Construction of various deletion plasmids

A hybrid plasmid (designated as pCAR16) was constructed by inserting a 1-6009 fragment, which was obtained by deletion to nucleotide position 6010 from the HindIII site (right terminal in Fig. 7) of the 6918 bp fragment containing the yellow pigment-synthesizing genes (DNA strand) (Fig. 7) using Deletion kit for kilo-sequence. pCAR16 contains the genes from crtE, crtX, crtY, crtI,crtB and crtZ. Various deletion plasmids were constructed, as shown in Table 1, on the basis of the pCAR16 and the aforementioned hybrid plasmid pCAR25 (containing genes crtE, crtX, crtY, crtI, crtB and crtZ.

### Table 1: Construction of various deletion plasmids

The number within parentheses behind the name of respective restriction enzymes represents the number of base at the initial recognition site of the restriction enzyme. The base numbers correspond to those in Figs. 1 - 6 and Fig. 7. Analysis of the metabolic intermediates of carotenoids was performed using the transformants of E. coli JM109 by various deletion plasmids [referred to hereinafter as E. coli (name of plasmid)].

**Table 1**

| Plasmid | Construction method | Genes functioning |
|---|---|---|
| pCAR25 | See text | crtE crtX crtY crtI crtB crtZ |
| pCAR25delB | Frame shift in BstEII (1235) of pCAR25 | crtE crtY crtI crtB crtZ |
| pCAR16 | See text | crtE crtX crtY crtI crtB |
| pCAR16delB | Frame shift in BstEII (1235) of pCAR16 | crtE crtY crtI crtB |
| pCAR16delC | Frame shift in SnaBI (3497) of pCAR16 | crtE crtX crtI crtB |
| pCAR-ADE | Deletion of the BstEII (1235) - SnaBI (3497) fragment from pCAR16 | crtE crtI crtB |
| pCAR-ADEF | Deletion of the BstEII (1235) - SnaBI (3497) fragment from pCAR25 | crtE crtI crtB crtZ |
| pCAR25delD | Frame shift in BamHI (3652) of pCAR25 | crtE crtX crtY crtB crtZ |
| pCAR-AE | Deletion of the BstEII (1235) - BamHI (3652) fragment from pCAR16 | crtE crtB |
| pCAR-A | Insertion of the KpnI (1) -BstEII (1235) fragment in pUC19 | crtE |
| pCAR-E | Insertion of the Eco52I (4926) - 6009 fragment in pUC19 | crtB |
| pCAR25delE | Frame shift in MluI (5379) of pCAR25 | crtE crtX crtY crtI crtZ |
| pCAR25delA | Frame shift in AvaI (995) of pCAR25 | crtX crtY crtI crtB crtZ |
| pCAR-CDE | Insertion of the SalI (2295) - 6009 fragment in pUC19 | crtY crtI crtB |

### (2) Identification of zeaxanthin

The cells harvested from 3 liters of 2 × YT medium of E. coli (pCAR25delB) (exhibiting orange) were extracted twice with 400 ml portions of acetone at low temperature, concentrated, then extracted with chloroform:methanol (9:1) and evaporated to dryness. This was subjected to silica gel column chromatography [30 cm × 3.0 cm (⌀)]. After the column was washed with chloroform, an orange band was eluted with chloroform:methanol (100:1). This pigment was dissolved in ethanol, recrystallized at low temperature to give 8 mg of a pure product. The analysis by its UV-visible absorption, ¹H-NMR, ¹³C-NMR and FD-MS (m/e 568) spectra revealed that this substance had the same structure except for stereochemistry as zeaxanthin (β,β-carotene-3,3′-diol). It was then dissolved in diethyl ether : isopentane : ethanol (5:5:2), and the CD spectrum was measured. As a result, it was found that this substance had a 3R,3′R-stereochemistry [Phytochemistry, 27, 3605-3609 (1988)]. Therefore, it was identified as zeaxanthin (β,β-carotene-3R,3′R-diol), of which the structure is illustrated below. The yield was 2.2 mg/g dry weight. This substance corresponded to the yellow pigment having an Rf value of 0.93 in Experimental Example (1).

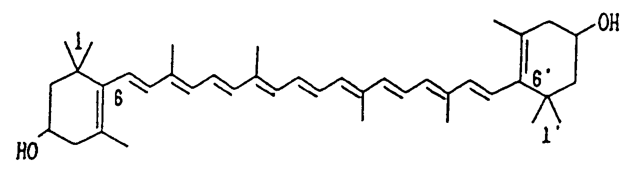

### (3) Identification of β-carotene

The cells harvested from 3 liters of LB medium of E. coli (pCAR16) (exhibiting orange) were extracted 3 times with 500 ml portions of cold methanol at low temperature and the methanol extract was further extracted with 1.5 liter of hexane. The hexane layer was concentrated and subjected to silica gel column chromatography [30 cm × 3.0 cm (⌀)]. Development and elution were conducted with hexane:ethyl acetate (50:1) to collect an orange band. The orange fraction was concentrated and recrystallized from ethanol to give 8 mg (reduced weight without moisture). This substance was presumed to belong to β-carotene from its UV-visible absorption spectrum, and a molecular weight of 536 by FD-MS spectrum also supported this presumption. Upon comparing this substance with the authentic sample (Sigma) of β-carotene by ¹³C-NMR spectrum, all of chemical shifts of carbons were identical with each other. Thus, this substance was identified as β-carotene (all-trans-β,β-carotene, of which the structure was illustrated below). It was also confirmed by the similar method that E. coli (pCAR16delB) accumulated the same β-carotene as described above. Its yield was 2.0 mg/g dry weight, which corresponded to 2 - 8 times (per dry weight) of the total carotenoid yield in carrot (Kintokininjin) culture cells described in Soshikibaiyou (The Tissue Culture), 13, 379-382 (1987).

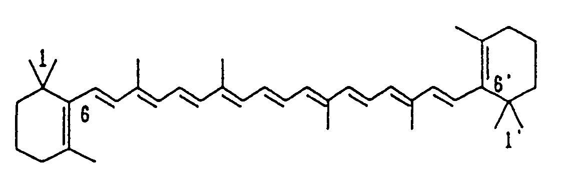

### (4) Identification of lycopene

The cells harvested from 3 liters of LB medium of E. coli (pCAR16delC) (exhibiting red) were extracted once with 500 ml of cold methanol at low temperature, and the precipitate by centrifugation was extracted again with 1.5 liter of chloroform. The chloroform layer was concentrated and subjected to silica gel chromatography [30 cm × 3.0 cm (⌀)]. Development and elution were conducted with hexane:chloroform (1:1) to collect a red band. This fraction was concentrated. This substance was presumed to belong to lycopene from its UV-visible absorption spectrum, and a molecular weight of 536 by FD-MS spectrum also supported this presumption. Upon comparing this substance with the authentic sample (Sigma) of lycopene by ¹H-NMR spectrum, all of chemical shifts of hydrogens were identical with each other. When, this substance and the authentic sample were subjected to TLC with silica gel 60 (Merck) [developed with hexane:chloroform (50:1)] and with RP-18 [developed with methanol:chloroform (4:1)], the displacement distances of these samples were completely equal to each other. Thus this substance was identified as lycopene (all-trans-β,β-carotene, of which the structure was illustrated below). It was also confirmed by the similar method that E. coli (pCAR-ADE) and E. coli (pCAR-ADEF) accumulated the same lycopene as described above. The yield of the former was 2.0 mg/g dry weight, which corresponded to 2 times (per dry weight) of the total carotenoid yield in a hyperproduction derivative of carrot (Kintokininjin) culture cells described in Soshikibaiyou (The Tissue Culture), 13, 379-382 (1987).

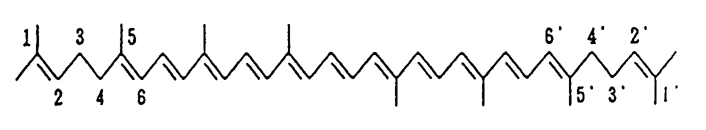

### (5) Identification of phytoene

The cells harvested from 1.5 liter of 2 × YT medium of E. coli (pCAR-AE) were extracted twice with 200 ml portions of acetone and twice with 100 ml portions of hexane, and evaporated to dryness. This was subjected to silica gel chromatography [30 cm × 3.0 cm (⌀)]. Development and elution were conducted with hexane:chloroform (1:1) to collect a band which had a strong UV absorption, and it was confirmed to be phytoene by its UV absorption spectrum. It was further subjected to LH-20 column chromatography [30 cm × 3.0 cm (⌀)]. Development and elution were conducted with chloroform:methanol (1:1) to give 4 mg of a pure product. The comparison of the ¹H-NMR spectrum of this substance with the ¹H-NMR spectra of trans- and cis-phytoen (J. Magnetic Resonance, 10, 43-50 (1973)) showed this substance to be a mixture of the trans- and cis-isomers. Isomerization from trans-isomer to cis-isomer hardly occurs, and thus it was judged that such a mixture was produced as a result of cis-trans isomerization in the course of the purification. Therefore, it was concluded that the original phytoene was the cis- -type phytoene (15,15'-cis-phytoene, of which the structure is shown below). It was also confirmed by the similar method that E. coli (pCAR25delD) accumulated the same phytoene as described above.

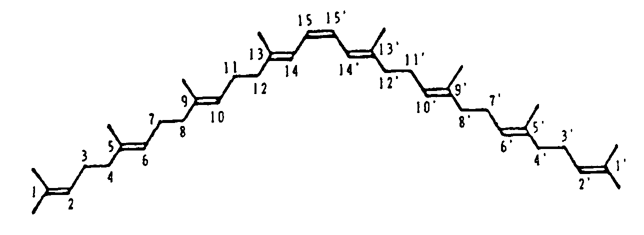

### Experimental Example 5: Identification of carotenoid biosynthesis genes

From the facts that E. coli (pCAR25) produced zeaxanthin-diglucoside and that E. coli (pCAR25delB) harboring a plasmid, in which crtX had been removed from pCAR25, accumulated zeaxanthin, it was found that the crtX gene encoded the glycosylation enzyme which was capable of converting zeaxanthin into zeaxanthin-diglucoside. Similarly, from the fact that E. coli (pCAR16delB) harboring a plasmid, in which crtZ had been removed from pCAR25delB, accumulated β-carotene, it was found that the crtZ gene encoded the hydroxylation enzyme which was capable of converting β-carotene into zeaxanthin. Similarly, from the fact that the E. coli (pCAR-ADE) harboring a plasmid, in which crtY had been removed from pCAR16delB, accumulated lycopene, it was found that the crtY gene encoded the cyclization enzyme which was capable of converting lycopene into β-carotene. Also, E. coli (pCAR-ADEF) carrying both of the crtE, crtI and crtB genes required for producing lycopene and the crtZ gene encoding the hydroxylation enzyme was able to synthesize only lycopene. This demonstrates directly that the hydroxylation reaction in carotenoid biosynthesis occurs after the cyclization reaction. Further, from the facts that E. coli (pCAR-ADE) accumulated lycopene and that E. coli (pCAR-AE) harboring a plasmid, in which the crtI gene had been removed from pCAR-ADE, accumulated phytoene, it was found that the crtI gene encoded the desaturation enzyme which was capable of converting phytoene into lycopene. E. coli (pCAR-A) and E. coli (pCAR-E) were not able to produce phytoene. It was thought from this result that both of the crtE and crtB genes were required for producing phytoene in E. coli. crtB and crtE were identified as the gene for the conversion of geranylgeranyl pyrophosphate into prephytoene pyrophosphate and that for the conversion of prephytoene pyrophosphate into phytoene, by comparing their putative amino acid sequence with those of crtB and crtE gene products in a photo synthetic bacterium Rhodobacter capsuratus [Mol. Gen. Genet., 216, 254-268 (1988)]. From these analyses described above, all of the six crt genes have been identified and the biosynthetic pathway of carotenoids have also been clear. These results are listed in Fig. 8.

E. coli (pCAR25delE) accumulated no detectable carotenoid intermediate, while E. coli (pCAR25delA) and E. coli (pCAR-CDE) were able to produce a small amount of carotenoids. That is to say, E. coli (pCAR25delA) and E. coli (pCAR-CDE) produced 4% of zeaxanthin-diglucoside and 2% of β-carotene as compared with the E. coli (pCAR25) and the E. coli (pCAR16delB), respectively. This result suggests that the reaction from prephytoene pyrophosphate to phytoene may occur non-enzymatically notwithstanding the yield being trace.

As described above, the detailed biosynthetic pathway of carotenoids including general and famous carotenoids such as lycopene, β-carotene and zeaxanthin and water soluble carotenoid such as zeaxanthin-diglucoside were for the first time elucidated, and the gene cluster useful for these biosynthesis was capable of being acquired for the first time. In this connection, lycopene, β-carotene and zeaxanthin which were produced by the genes in the aforementioned Experimental Examples were stereochemically identical with those derived from higher plants [T.W. Goodwin: "Plant Pigments", Academic Press (1988)].

As for zeaxanthin-diglucoside, the isolation from a plant was only reported [Pure & Appl. Chem., 47, 121-128 (1976)], but its isolation from microorganisms has not been reported.

### Experimental Example 6: Synthesis of carotenoids in Zymomonas

Zymomonas mobilis is a facultative anaerobic ethanol-producing bacterium. It has a higher ethanol producing rate than that of yeast (Saccharomyces cerevisiae), so that it is preferable as a fuel alcohol-producing bacterium in future. Also, Zymomonas has a special metabolic pathway, Entner-Doudoroff but not glycolytic pathway and cannot produce carotenoids. In order to add further values to this bacterium, the carotenoid biosynthesis genes were introduced into Zymomonas.

The 7.6 kb fragment containing the DNA sequence shown in Fig. 7 was cut out from the hybrid plasmid pCAR1 by KpnI digestion and treated with DNA polymerase I (Klenow enzyme). The fragment thus treated was ligated to the EcoRV site of the cloning vector pZA22 for Zymomonas [see Agric. Biol. Chem., 50, 3201-3203 (1986) and Japanese Patent Laid-Open Publication No. 228278/87] to construct a hybrid plasmid pZACAR1. Also, the 1 - 6009 fragment in the DNA sequence in Fig. 7 was cut out from pCAR16 by KpnI/EcoRI digestion and treated with DNA polymerase I (Klenow enzyme). The fragment thus treated was ligated to the EcoRV site of pZA22 to construct a hybrid plasmid pZACAR16. The orientation of the inserted fragments in these plasmids were opposite with the orientation of the Tc^{r} gene on taking the orientation in Fig. 7 as the normal one. These plasmids were introduced into Z. mobilis NRRL B-14023 by conjugal transfer with the helper plasmid pRK2013 (ATCC 37159) and stably maintained in this strain. Z. mobilis NRRL B-14023 in which pZACAR1 and pZACAR16 had been introduced exhibited yellow, and produced zeaxanthin-diglucoside in an amount of 0.28 mg/g dry weight and β-carotene in an amount of 0.14 mg/g dry weight, respectively. Therefore, carotenoids were successfully produced in Zymomonas by the carotenoid biosynthesis genes according to the present invention.

### Deposition of Microorganism

Microorganism relating to the present invention is deposited at Fermentation Research Institute, Japan as follows:

| Microorganism | Accession number | Date of deposit |
|---|---|---|
| Escherichia coli JM109 (pCAR1) | FERM BP 2377 | April 11, 1989 |

## Claims

1. A DNA sequence selected from the following group and encoding an enzyme polypeptide which participates in carotenoid biosynthesis proceeding via geranylgeranyl pyrophosphate, phytoene and zeaxanthin-diglucoside:
a DNA sequence encoding an enzyme polypeptide which paticipates in a step before the phytoene stage in the carotenoid biosynthesis and whose amino acid sequence corresponds substantially to the amino acid sequence from A to B shown in Figs. 1(a) and (b);
a DNA sequence encoding a polypeptide which has the enzymatic activity of converting zeaxanthin into zeaxanthin-diglucoside in the carotenoid biosynthesis and whose amino acid sequence corresponds substantially to the amino acid sequence from C to D shown in Figs. 2(a) and (b);
a DNA sequence encoding a polypeptide which has the enzymatic activity of converting lycopene into β-carotene in the carotenoid biosynthesis and whose amino acid sequence corresponds substantially to the amino acid sequence from E to F shown in Figs. 3(a) and (b);
a DNA sequence encoding a polypeptide which has the enzymatic activity of converting phytoene into lycopene in the carotenoid biosynthesis and whose amino acid sequence corresponds substantially to the amino acid sequence from G to H shown in Figs. 4(a),(b) and (c);
a DNA sequence encoding a polypeptide which has the enzymatic activity of converting geranylgeranyl pyrophosphate as a substrate into a next carotenoid compound in the carotenoid biosynthesis and whose amino acid sequence corresponds substantially to the amino acid sequence from I to J shown in Figs. 5(a) and (b); and
a DNA sequence encoding a polypeptide which has the enzymatic activity of converting β-carotene into zeaxanthin in the carotenoid biosynthesis and whose amino acid sequence corresponds substantially to the amino acid sequence from K to L shown in Figs. 6.

2. A DNA sequence according to claim 1, which encodes an enzyme polypeptide participating in a step before the phytoene stage in the carotenoid biosynthesis and having the amino acid sequence corresponding substantially to the amino acid sequence from A to B shown in Figs. 1(a) and (b).

3. A DNA sequence according to claim 1, which encodes a polypeptide having the enzymatic activity of converting zeaxanthin into zeaxanthin-diglucoside in the carotenoid biosynthesis and having the amino acid sequence corresponding substantially to the amino acid sequence from C to D shown in Figs. 2(a) and (b).

4. A DNA sequence according to claim 1, which encodes a polypeptide having the enzymatic activity of converting lycopene into β-carotene in the carotenoid biosynthesis and having the amino acid sequence corresponding substantially to the amino acid sequence from E to F shown in Figs. 3(a) and (b).

5. A DNA sequence according to claim 1, which encodes a polypeptide having the enzymatic activity of converting phytoene into lycopene in the carotenoid biosynthesis and having the amino acid sequence corresponding substantially to the amino acid sequence from G to H shown in Figs. 4(a),(b) and (c).

6. A DNA sequence according to claim 1, which encodes a polypeptide having the enzymatic activity of converting geranylgeranyl pyrophosphate as a substrate into a next carotenoid compound in the carotenoid biosynthesis and having the amino acid sequence corresponding substantially to the amino acid sequence from I to J shown in Figs. 5(a) and (b).

7. A DNA sequence according to claim 1, which encodes a polypeptide having the enzymatic activity of converting β-carotene into zeaxanthin in the carotenoid biosynthesis and having the amino acid sequence corresponding substantially to the amino acid sequence from K to L shown in Figs. 6.

8. Process for producing a carotenoid or a precursor compound which is selected from the group consisting of phytoene, lycopene, β-carotene, zeaxanthin and zeaxanthin-diglucoside, characterised in that a host is transformed with at least one of the DNAs selected from the following DNA sequences encoding the enzyme polypeptides which participate in carotenoid biosynthesis proceeding via geranylgeranyl pyrophosphate, phytoene and zeaxanthin-diglucoside:
a DNA sequence encoding an enzyme polypeptide which participates in the step before the phytoene stage in the carotenoid biosynthesis and whose amino acid sequence corresponds substantially to the amino acid sequence from A to B shown in Figs. 1(a) and (b);
a DNA sequence encoding a polypeptide which has the enzymatic activity of converting zeaxanthin into zeaxanthin-diglucoside in the carotenoid biosynthesis and whose amino acid sequence corresponds substantially to the amino acid sequence from C to D shown in Figs. 2(a) and (b);
a DNA sequence encoding a polypeptide which has the enzymatic activity of converting lycopene into β-carotene in the carotenoid biosynthesis and whose amino acid sequence corresponds substantially to the amino acid sequence from E to F shown in Figs. 3(a) and (b);
a DNA sequence encoding a polypeptide which has the enzymatic activity of converting phytoene into lycopene in the carotenoid biosynthesis and whose amino acid sequence corresponds substantially to the amino acid sequence from G to H shown in Figs. 4(a),(b) and (c);
a DNA sequence encoding a polypeptide which has the enzymatic activity of converting geranylgeranyl pyrophosphate as a substrate into a next carotenoid compound in the carotenoid biosynthesis and whose amino acid sequence corresponds substantially to the amino acid sequence from I to J shown in Figs. 5(a) and (b); and
a DNA sequence encoding a polypeptide which has the enzymatic activity of converting β-carotene into zeaxanthin in the carotenoid biosynthesis and whose amino acid sequence corresponds substantially to the amino acid sequence from K to L shown in Figs. 6;
and the transformant is cultured.

## Patentansprüche

1. DNS-Sequenz, die aus der folgenden Gruppe ausgewählt ist und ein Enzym-Polypeptid codiert, das an der über Geranylgeranylpyrophosphat, Phytoen und Zeaxanthindiglucosid verlaufenden Carotinoid-Biosynthese beteiligt ist:
DNS-Sequenz, die ein Enzym-Polypeptid codiert, das bei der Carotinoid-Biosynthese auf einer Stufe vor der Phytoenstufe beteiligt ist und dessen Aminosäuresequenz im wesentlichen der in Fig. 1(a) und (b) von A bis B dargestellten Aminosäuresequenz entspricht;
DNS-Sequenz, die ein Polypeptid codiert, das die enzymatische Aktivität der Umwandlung von Zeaxanthin in Zeaxanthindiglucosid bei der Carotinoid-Biosynthese besitzt und dessen Aminosäuresequenz im wesentlichen der in FIG.2(a) und (b) von C bis D dargestellten Aminosäuresequenz entspricht;
DNS-Sequenz, die ein Polypeptid codiert, das die enzymatische Aktivität der Umwandlung von Lycopin in β-Carotin bei der Carotinoid-Biosynthese aufweist und dessen Aminosäuresequenz im wesentlichen der in Fig. 3(a) und (b) von E bis F dargestellten Aminosäuresequenz entspricht;
DNS-Sequenz, die ein Polypeptid codiert, das die enzymatische Aktivität der Umwandlung von Phytoen in Lycopin bei der Carotinoid-Biosynthese aufweist und dessen Aminosäuresequenz im wesentlichen der in Fig. 4(a), (b) und (c) von G bis H dargestellten Aminosäuresequenz entspricht;
DNS-Sequenz, die ein Polypeptid codiert, das die enzymatische Aktivität der Umwandlung von Geranylgeranylpyrophosphat bei der Carotinoid-Biosynthese in die nächste Carotinoid-Verbindung aufweist und dessen Aminosäuresequenz im wesentlichen der in Fig. 5(a) und (b) von I bis J dargestellten Aminosäuresequenz entspricht; und
DNS-Sequenz, die ein Polypeptid codiert, das die enzymatische Aktivität der Umwandlung von β-Carotin in Zeaxanthin bei der Carotinoid-Biosynthese aufweist und dessen Aminosäuresequenz im wesentlichen der in Fig. 6 von K bis L dargestellten Aminosäuresequenz entspricht.

2. DNS-Sequenz gemäß Anspruch 1, die ein Enzym-Polypeptid codiert, das in der Carotinoid-Biosynthese auf einer Stufe vor der Phytoenstufe beteiligt ist und eine Aminosäuresequenz aufweist, die im wesentlichen der in Fig. 1(a) und (b) von A bis B dargestellten Aminosäuresequenz entspricht.

3. DNS-Sequenz gemäß Anspruch 1, die ein Polypeptid codiert, das die enzymatische Aktivität der Umwandlung von Zeaxanthin in Zeaxanthindiglucosid bei der Carotinoid-Biosynthese besitzt und eine Aminosäuresequenz aufweist, die im wesentlichen der in Fig. 2(a) und (b) von C bis D dargestellten Aminosäuresequenz entspricht.

4. DNS-Sequenz gemäß Anspruch 1, die ein Polypeptid codiert, das die enzymatische Aktivität der Umwandlung von Lycopin in β-Carotin bei der Carotinoid-Biosynthese besitzt und eine Aminosäuresequenz aufweist, die im wesentlichen der in Fig. 3(a) und (b) von E bis F dargestellten Aminosäuresequenz entspricht.

5. DNS-Sequenz gemäß Anspruch 1, die ein Polypeptid codiert, das die enzymatische Aktivität der Umwandlung von Phytoen in Lycopin bei der Carotinoid-Biosynthese besitzt und eine Aminosäuresequenz aufweist, die im wesentlichen der in Fig. 4(a), (b) und (c) von G bis H dargestellten Aminosäuresequenz entspricht.

6. DNS-Sequenz gemäß Anspruch 1, die ein Polypeptid codiert, das die enzymatische Aktivität der Umwandlung von Geranylgeranylpyrophosphat als Substrat bei der Carotinoid-Biosynthese in die nächste Carotinoidverbindung besitzt und eine Aminosäuresequenz aufweist, die im wesentlichen der in Fig. 5(a) und (b) von I bis J dargestellten Aminosäuresequenz entspricht.

7. DNS-Sequenz gemäß Anspruch 1, die ein Polypeptid codiert, das die enzymatische Aktivität der Umwandlung von β-Carotin in Zeaxanthin bei der Carotinoid-Biosynthese besitzt und eine Aminosäuresequenz aufweist, die im wesentlichen der in Fig. 6 von K bis L dargestellten Aminosäuresequenz entspricht.

8. Verfahren zur Herstellung eines Carotinoids oder einer Vorläuferverbindung aus der Gruppe Phytoen, Lycopin, β-Carotin, Zeaxanthin und Zeaxanthindiglucosid,
**dadurch gekennzeichnet,**
daß ein Wirt mit mindestens einer der aus den folgenden DNS-Sequenzen ausgewählten Desoxyribonucleinsäuren transformiert wird, die die Enzym-Polypeptide codieren, die bei der über Geranylgeranylpyrophosphat, Phytoen und Zeaxanthindiglucosid verlaufenden Carotinoid-Biosynthese beteiligt sind:
DNS-Sequenz, die ein Enzym-Polypeptid codiert, das bei der Carotinoid-Biosynthese auf einer Stufe vor der Phytoenstufe beteiligt ist und dessen Aminosäuresequenz im wesentlichen der in Fig. 1(a) und (b) von A bis B dargestellten Aminosäuresequenz entspricht;
DNS-Sequenz, die ein Polypeptid codiert, das die enzymatische Aktivität der Umwandlung von Zeaxanthin in Zeaxanthindiglucosid bei der Carotinoid-Biosynthese besitzt und dessen Aminosäuresequenz im wesentlichen der in FIG.2(a) und (b) von C bis D dargestellten Aminosäuresequenz entspricht;
DNS-Sequenz, die ein Polypeptid codiert, das die enzymatische Aktivität der Umwandlung von Lycopin in β-Carotin bei der Carotinoid-Biosynthese aufweist und dessen Aminosäuresequenz im wesentlichen der in Fig. 3(a) und (b) von E bis F dargestellten Aminosäuresequenz entspricht;
DNS-Sequenz, die ein Polypeptid codiert, das die enzymatische Aktivität der Umwandlung von Phytoen in Lycopin bei der Carotinoid-Biosynthese aufweist und dessen Aminosäuresequenz im wesentlichen der in Fig. 4(a), (b) und (c) von G bis H dargestellten Aminosäuresequenz entspricht;
DNS-Sequenz, die ein Polypeptid codiert, das die enzymatische Aktivität der Umwandlung von Geranylgeranylpyrophosphat bei der Carotinoid-Biosynthese in die nächste Carotinoid-Verbindung aufweist und dessen Aminosäuresequenz im wesentlichen der in Fig. 5(a) und (b) von I bis J dargestellten Aminosäuresequenz entspricht; und
DNS-Sequenz, die ein Polypeptid codiert, das die enzymatische Aktivität der Umwandlung von β-Carotin in Zeaxanthin bei der Carotinoid-Biosynthese aufweist und dessen Aminosäuresequenz im wesentlichen der in Fig. 6 von K bis L dargestellten Aminosäuresequenz entspricht; und das Transformationsprodukt gezüchtet wird.

## Revendications

1. Séquence d'ADN choisie parmi l'ensemble ci-après, et codant un polypeptide enzymatique qui participe à la biosynthèse des caroténoïdes par l'intermédiaire du pyrophosphate de géranylgéranyle, du phytoène et du zéaxanthine-diglucoside :
une séquence d'ADN codant un polypeptide enzymatique qui participe à une étape préalable à la phase phytoène de la biosynthèse des caroténoïdes, et dont la séquence d'acides aminés correspond essentiellement à la séquence d'acides aminés allant de A à B sur les figures 1(a) et (b) ;
une séquence d'ADN codant un polypeptide qui a une activité enzymatique de conversion de la zéaxanthine en zéaxanthine-diglucoside dans la biosynthèse des caroténoïdes, et dont la séquence d'acides aminés correspond essentiellement à la séquence d'acides aminés allant de C à D sur les figures 2(a) et (b) ;
une séquence d'ADN codant un polypeptide qui a une activité enzymatique de conversion du lycopène en β-carotène dans la biosynthèse des caroténoïdes et dont la séquence d'acides aminés correspond essentiellement à la séquence d'acides aminés allant de E à F sur les figures 3(a) et (b) ;
une séquence d'ADN codant un polypeptide qui a une activité enzymatique de conversion du phytoène en lycopène dans la biosynthèse des caroténoïdes, et dont la séquence d'acides aminés correspond essentiellement à la séquence d'acides aminés allant de G à H sur les figures 4(a), (b) et (c) ;
une séquence d'ADN codant un polypeptide qui a une activité enzymatique de conversion du pyrophosphate de géranylgéranyle servant de substrat en un composé caroténoïde suivant dans la biosynthèse des caroténoïdes et dont la séquence d'acides aminés correspond essentiellement à la séquence d'acides aminés allant de I à J sur les figures 5(a) et (b) ; et
une séquence d'ADN codant un polypeptide qui a une activité enzymatique de conversion du β-carotène en zéaxantine dans la biosynthèse des caroténoïdes et dont la séquence d'acides aminés correspond essentiellement à la séquence d'acides aminés allant de K à L sur la figure 6.

2. Séquence d'ADN selon la revendication 1, qui code un polypeptide enzymatique qui participe à une étape antérieure à la phase phytoène de la biosynthèse des caroténoïdes, et dont la séquence d'acides aminés correspond essentiellement à la séquence d'acides aminés allant de A à B sur les figures 1(a) et (b).

3. Séquence d'ADN selon la revendication 1, qui code un polypeptide ayant une activité enzymatique de conversion de la zéaxantine en zéaxantine-diglucoside dans la biosynthèse des caroténoïdes et dont la séquence d'acides aminés correspond essentiellement à la séquence d'acides aminés allant de C à D sur les figures 2(a) et (b).

4. Séquence d'ADN selon la revendication 1, qui code un polypeptide ayant une activité enzymatique de conversion du lycopène en β-carotène dans la biosynthèse des caroténoïdes et dont la séquence d'acides aminés correspond essentiellement à la séquence d'acides aminés allant de E à F sur les figures 3(a) et (b).

5. Séquence d'ADN selon la revendication 1, qui code un polypeptide ayant une activité enzymatique de conversion du phytoène en lycopène dans la biosynthèse des caroténoïdes, et dont la séquence d'acides aminés correspond essentiellement à la séquence d'acides aminés allant de G à H sur les figures 4(a), (b) et (c).

6. Séquence d'ADN selon la revendication 1, qui code un polypeptide ayant une activité enzymatique de conversion du pyrophosphate de géranylgéranyle servant de substrat en un composé caroténoïde suivant dans la biosynthèse des caroténoïdes et dont la séquence d'acides aminés correspond essentiellement à la séquence d'acides aminés allant de I à J sur les figures 5(a) et (b).

7. Séquence d'ADN selon la revendication 1, qui code un polypeptide ayant une activité enzymatique de conversion du β-carotène en zéaxantine dans la biosynthèse des caroténoïdes et dont la séquence d'acides aminés correspond essentiellement à la séquence d'acides aminés allant de K à L sur la figure 6.

8. Procédé pour produire un caroténoïde ou un précurseur choisi parmi l'ensemble comprenant le phytoène, le lycopène, le β-carotène, la zéaxanthine et le zéaxantine-diglucoside, caractérisé en ce qu'on transforme un hôte avec au moins l'un des ADN choisis parmi les séquences d'ADN ci-après codant les polypeptides enzymatiques qui participent à la biosynthèse des caroténoïdes par l'intermédiaire du pyrophosphate de géranylgéranyle, du phytoène et du zéaxantine-diglucoside :
une séquence d'ADN codant un polypeptide enzymatique qui participe à une étape préalable à la phase phytoène de la biosynthèse des caroténoïdes, et dont la séquence d'acides aminés correspond essentiellement à la séquence d'acides aminés allant de A à B sur les figures 1(a) et (b) ;
une séquence d'ADN codant un polypeptide qui a une activité enzymatique de conversion de la zéaxanthine en zéaxanthine-diglucoside dans la biosynthèse des caroténoïdes, et dont la séquence d'acides aminés correspond essentiellement à la séquence d'acides aminés allant de C à D sur les figures 2(a) et (b) ;
une séquence d'ADN codant un polypeptide qui a une activité enzymatique de conversion du lycopène en β-carotène dans la biosynthèse des caroténoïdes et dont la séquence d'acides aminés correspond essentiellement à la séquence d'acides aminés allant de E à F sur les figures 3(a) et (b) ;
une séquence d'ADN codant un polypeptide qui a une activité enzymatique de conversion du phytoène en lycopène dans la biosynthèse des caroténoïdes, et dont la séquence d'acides aminés correspond essentiellement à la séquence d'acides aminés allant de G à H sur les figures 4(a), (b) et (c) ;
une séquence d'ADN codant un polypeptide qui a une activité enzymatique de conversion du pyrophosphate de géranylgéranyle servant de substrat en un composé caroténoïde suivant dans la biosynthèse des caroténoïdes et dont la séquence d'acides aminés correspond essentiellement à la séquence d'acides aminés allant de I à J sur les figures 5(a) et (b) ; et
une séquence d'ADN codant un polypeptide qui a une activité enzymatique de conversion du β-carotène en zéaxantine dans la biosynthèse des caroténoïdes et dont la séquence d'acides aminés correspond essentiellement à la séquence d'acides aminés allant de K à L sur la figure 6 ;
et en ce que l'on cultive le transformant.
